# EUROPEAN PATENT SPECIFICATION

(11) **EP 2 316 936 B1**
(45) Date of publication and mention of the grant of the patent: **07.08.2013**
(21) Application number: 09758131.8
(22) Date of filing: 04.06.2009
(51) Int. Cl.: C12N 15/09, C12P 21/08, C12Q 1/02, C12Q 1/68, G01N 33/68

(54) **METHOD FOR ANALYSIS/IDENTIFICATION OF ANTIBODY GENE AT ONE-CELL LEVEL**
VERFAHREN ZUR ANALYSE/IDENTIFIZIERUNG EINES ANTIKÖRPERGENS AUF EINZELZELLEBENE
PROCÉDÉ D'ANALYSE/IDENTIFICATION D'UN GÈNE D'ANTICORPS À UN NIVEAU UNICELLULAIRE

(30) Priority: 04.06.2008 JP 2008146964
(43) Date of publication of application: 04.05.2011
(73) Proprietor: Shizuoka Prefecture, Shizuoka 420-8601 (JP)
(72) Inventor: AKIYAMA, Yasuto, Sunto-gun Shizuoka 411-8777 (JP)
(74) Representative: Richards, William John
(86) International application number: PCT/JP2009/002539
(87) International publication number: WO 2009/147864

(56) References cited:
- WO-A1-00/20460
- WO-A1-2004/087914
- WO-A2-2007/001420
- JP-A- 2004 121 237
- JP-A- 2008 295 415
- US-A- 5 256 542
- US-A1- 2007 122 852
- LIEBY P. ET AL.: 'Memory B cells producing somatically mutated antiphospholipid antibodies are present in healthy individuals' BLOOD vol. 102, no. 7, 01 October 2003, pages 2459 - 2465, XP008138613
- TILLER T. ET AL.: 'Efficient generation of monoclonal antibodies from single human B cells by single cell RT-PCR and expression vector cloning' J. IMMUNOL. METHODS vol. 329, no. 1-2, 01 January 2008, pages 112 - 124, XP022389335
- VOLKHEIMER A.D. ET AL.: 'Progressive immunoglobulin gene mutations in chronic lymphocytic leukemia: evidence for antigen-driven intraclonal diversification' BLOOD vol. 109, no. 4, 15 February 2007, pages 1559 - 1567, XP009136340
- WRAMMERT J. ET AL.: 'Rapid cloning of high-affinity human monoclonal antibodies against influenza virus' NATURE vol. 453, no. 7195, 29 May 2008, pages 667 - 672, XP002524388
- ROMERO P. ET AL.: 'Antigenicity and immunogenicity of Melan-A/MART-1 derived peptides as targets for tumor reactive CTL in human melanoma' IMMUNOL. REV. vol. 188, October 2002, pages 81 - 96, XP009143135

### SEQUENCE LISTING

<110> Shizuoka prefecture
<120> Methods for determining human antibody gene derived from single B cell
<130> 2009C5175
<150> JP 2008-146964
   <151> 2008-06-04
<160> 86
<170> PatentIn version 3.1
<210> 1
   <211> 27
   <212> DNA
   <213> Artificial
<220>
   <223> HuIGHV_1
<400> 1
   ctcaccatgg actggacctg gaggatc 27
<210> 2
   <211> 27
   <212> DNA
   <213> Artificial
<220>
   <223> HuIGHV_2
<400> 2
   ctcaccatgg actggacctg gagcatc 27
<210> 3
   <211> 27
   <212> DNA
   <213> Artificial
<220>
   <223> HuIGHV_3
<400> 3
   ctcaccatgg actgcacctg gaggatc 27
<210> 4
   <211> 27
   <212> DNA
   <213> Artificial
<220>
   <223> HuIGHV_4
<400> 4
   ttcaccatgg actggacctg gagggtc 27
<210> 5
   <211> 27
   <212> DNA
   <213> Artificial
<220>
   <223> HuIGHV_5
<400> 5
   ctcactatgg actggatttg gagggtc 27
<210> 6
   <211> 27
   <212> DNA
   <213> Artificial
<220>
   <223> HuIGHV_6
<400> 6
   atcaccatgg actggacctg gaggttc 27
<210> 7
   <211> 27
   <212> DNA
   <213> Artificial
<220>
   <223> HuIGHV_7
<400> 7
   cccaccatgg acacactttg ctccacg 27
<210> 8
   <211> 27
   <212> DNA
   <213> Artificial
<220>
   <223> HuIGHV_8
<400> 8
   cccaccatgg acacactttg ctacaca 27
<210> 9
   <211> 27
   <212> DNA
   <213> Artificial
<220>
   <223> HuIGHV_9
<400> 9
   tccaccatgg acatactttg ttccacg 27
<210> 10
   <211> 27
   <212> DNA
   <213> Artificial
<220>
   <223> HuIGHV_10
<400> 10
   ctcaccatgg aattggggct gagctgg 27
<210> 11
   <211> 27
   <212> DNA
   <213> Artificial
<220>
   <223> HuIGHV_11
<400> 11
   ctcaccatgg agtttgggct gagctgg 27
<210> 12
   <211> 27
   <212> DNA
   <213> Artificial
<220>
   <223> HuIGHV_12
<400> 12
   attaccatgg aatttgggct gagctgg 27
<210> 13
   <211> 27
   <212> DNA
   <213> Artificial
<220>
   <223> HuIGHV_13
<400> 13
   ttcaccatgg aactggggct ccgctgg 27
<210> 14
   <211> 27
   <212> DNA
   <213> Artificial
<220>
   <223> HuIGHV_14
<400> 14
   ctcatcatgc agtttgtgct gagctgg 27
<210> 15
   <211> 27
   <212> DNA
   <213> Artificial
<220>
   <223> HuIGHV_15
<400> 15
   cgcaccatgg agtttggact gagctgg 27
<210> 16
   <211> 27
   <212> DNA
   <213> Artificial
<220>
   <223> HuIGHV_16
<400> 16
   ctcaccatgg agttggggct gtgctgg 27
<210> 17
   <211> 27
   <212> DNA
   <213> Artificial
<220>
   <223> HuIGHV_17
<400> 17
   ccaaccatgg agtttgggct tagctgg 27
<210> 18
   <211> 27
   <212> DNA
   <213> Artificial
<220>
   <223> HuIGHV_18
<400> 18
   ctcaccatgg agttttggct gagctgg 27
<210> 19
   <211> 27
   <212> DNA
   <213> Artificial
<220>
   <223> HuIGHV_19
<400> 19
   gtcatgatgg agtttgggct gagctgg 27
<210> 20
   <211> 27
   <212> DNA
   <213> Artificial
<220>
   <223> HuIGHV_20
<400> 20
   ctcgccatgg agtttgggct gagctgg 27
<210> 21
   <211> 27
   <212> DNA
   <213> Artificial
<220>
   <223> HuIGHV_21
<400> 21
   aagaacatga aacacctgtg gttcttc 27
<210> 22
   <211> 27
   <212> DNA
   <213> Artificial
<220>
   <223> HuIGHV_22
<400> 22
   aagaaaatga agcacctgtg gttcttc 27
<210> 23
   <211> 27
   <212> DNA
   <213> Artificial
<220>
   <223> HuIGHV_23
<400> 23
   accatcatgg ggtcaaccgc catcctc 27
<210> 24
   <211> 27
   <212> DNA
   <213> Artificial
<220>
   <223> HuIGHV_24
<400> 24
   cagacaatgt ctgtctcctt cctcatc 27
<210> 25
   <211> 27
   <212> DNA
   <213> Artificial
<220>
   <223> HuIGHC_G1.2.3
<400> 25
   tcatttaccc ggagacaggg agaggct 27
<210> 26
   <211> 27
   <212> DNA
   <213> Artificial
<220>
   <223> HuIGHC_G4
<400> 26
   tcatttaccc agagacaggg agaggct 27
<210> 27
   <211> 28
   <212> DNA
   <213> Artificial
<220>
   <223> HuIGKV_1
<400> 27
   cacagcatgg acatgagggt ccccgctc 28
<210> 28
   <211> 28
   <212> DNA
   <213> Artificial
<220>
   <223> HuIGKV_2
<400> 28
   atagacatga gggtccccgc tcagctcc 28
<210> 29
   <211> 28
   <212> DNA
   <213> Artificial
<220>
   <223> HuIGKV_3
<400> 29
   cacagcatgg acatgagagt cctcgctc 28
<210> 30
   <211> 28
   <212> DNA
   <213> Artificial
<220>
   <223> HuIGKV_4
<400> 30
   ctcacaatga ggctccttgc tcagcttc 28
<210> 31
   <211> 28
   <212> DNA
   <213> Artificial
<220>
   <223> HuIGKV_5
<400> 31
   ctcacaatga ggctccctgc tcagctcc 28
<210> 32
   <211> 28
   <212> DNA
   <213> Artificial
<220>
   <223> HuIGKV_6
<400> 32
   ctcaccatga ggctccctgc tcagctcc 28
<210> 33
   <211> 28
   <212> DNA
   <213> Artificial
<220>
   <223> HuIGKV_7
<400> 33
   ggaaccatgg aagccccagc tcagcttc 28
<210> 34
   <211> 28
   <212> DNA
   <213> Artificial
<220>
   <223> HuIGKV_8
<400> 34
   ggaaccatgg aaaccccagc gcagcttc 28
<210> 35
   <211> 28
   <212> DNA
   <213> Artificial
<220>
   <223> HuIGKV_9
<400> 35
   agcaagatgg tgttgcagac ccaggtct 28
<210> 36
   <211> 28
   <212> DNA
   <213> Artificial
<220>
   <223> HuIGKV_10
<400> 36
   aaggttatgg ggtcccaggt tcacctcc 28
<210> 37
   <211> 28
   <212> DNA
   <213> Artificial
<220>
   <223> HuIGKV_11
<400> 37
   aggaagatgt tgccatcaca actcattg 28
<210> 38
   <211> 27
   <212> DNA
   <213> Artificial
<220>
   <223> HuKC-1
<400> 38
   ctaacactct cccctgttga agctctt 27
<210> 39
   <211> 33
   <212> DNA
   <213> Artificial
<220>
   <223> HuIGLV_1
<400> 39
   gccaccatgg cctggtcccc tctcttcctc acc 33
<210> 40
   <211> 33
   <212> DNA
   <213> Artificial
<220>
   <223> HuIGLV_2
<400> 40
   gccaccatgg cctggtctcc tctcctcctc act 33
<210> 41
   <211> 33
   <212> DNA
   <213> Artificial
<220>
   <223> HuIGLV_3
<400> 41
   gccaccatgg ccagcttccc tctcctcctc acc 33
<210> 42
   <211> 33
   <212> DNA
   <213> Artificial
<220>
   <223> HuIGLV_4
<400> 42
   gccaccatgg cctgggctcc tctgctcctc acc 33
<210> 43
   <211> 33
   <212> DNA
   <213> Artificial
<220>
   <223> HuIGLV_5
<400> 43
   gccaccatga cctgctcccc tctcctcctc acc 33
<210> 44
   <211> 33
   <212> DNA
   <213> Artificial
<220>
   <223> HuIGLV_6
<400> 44
   gccaccatgg cctgggctct gctgctcctc acc 33
<210> 45
   <211> 33
   <212> DNA
   <213> Artificial
<220>
   <223> HuIGLV_7
<400> 45
   gccaccatgg cctgggctct gctcctcctc agc 33
<210> 46
   <211> 33
   <212> DNA
   <213> Artificial
<220>
   <223> HuIGLV_8
<400> 46
   gccaccatga ccctgctgca ggtggatggg ctc 33
<210> 47
   <211> 33
   <212> DNA
   <213> Artificial
<220>
   <223> HuIGLV_9
<400> 47
   gccaccatgg cctggaccgc tctccttctg agc 33
<210> 48
   <211> 33
   <212> DNA
   <213> Artificial
<220>
   <223> HuIGLV_10
<400> 48
   gccaccatgg cctggacccc tctcctgctc ccc 33
<210> 49
   <211> 33
   <212> DNA
   <213> Artificial
<220>
   <223> HuIGLV_11
<400> 49
   gccaccatgg cctggacccc tctcctcctc agc 33
<210> 50
   <211> 33
   <212> DNA
   <213> Artificial
<220>
   <223> HuIGLV_12
<400> 50
   gccaccatgg cctggacccc tctctggctc act 33
<210> 51
   <211> 33
   <212> DNA
   <213> Artificial
<220>
   <223> HuIGLV_13
<400> 51
   gccaccatgg cctggaccgt tctcctcctc ggc 33
<210> 52
   <211> 33
   <212> DNA
   <213> Artificial
<220>
   <223> HuIGLV-14
<400> 52
   gccaccatgg catgggccac actcctgctc cca 33
<210> 53
   <211> 33
   <212> DNA
   <213> Artificial
<220>
   <223> HuIGLV_15
<400> 53
   gccaccatgg cctggatccc tctacttctc ccc 33
<210> 54
   <211> 33
   <212> DNA
   <213> Artificial
<220>
   <223> HuIGLV_16
<400> 54
   gccaccatgg cctgggtctc cttctaccta ctg 33
<210> 55
   <211> 33
   <212> DNA
   <213> Artificial
<220>
   <223> HuIGLV_17
<400> 55
   gccaccatgg cctggactcc tctcctcctc ctg 33
<210> 56
   <211> 33
   <212> DNA
   <213> Artificial
<220>
   <223> HuIGLV_18
<400> 56
   gccaccatgg cttggacccc actcctcttc ctc 33
<210> 57
   <211> 33
   <212> DNA
   <213> Artificial
<220>
   <223> HuIGLV_19
<400> 57
   gccaccatgg gaagaaaagg aggcctgggg cag 33
<210> 58
   <211> 33
   <212> DNA
   <213> Artificial
<220>
   <223> HuIGLV_20
<400> 58
   gccaccatgg agaagaagag gagacctggg gca 33
<210> 59
   <211> 33
   <212> DNA
   <213> Artificial
<220>
   <223> HuIGLV_21
<400> 59
   gccaccatgg cctgggctcc actacttctc acc 33
<210> 60
   <211> 33
   <212> DNA
   <213> Artificial
<220>
   <223> HuIGLV_22 .
<400> 60
   gccaccatgg cctggactcc tctctttctg ttc 33
<210> 61
   <211> 33
   <212> DNA
   <213> Artificial
<220>
   <223> HuIGLV_23
<400> 61
   gccaccatgg cctggatgat gcttctcctc gga 33
<210> 62
   <211> 28
   <212> DNA
   <213> Artificial
<220>
   <223> HuLC-1
<400> 62
   ctatgaacat tctgtagggg ccactgtc 28
<210> 63
   <211> 28
   <212> DNA
   <213> Artificial
<220>
   <223> HuLC-2
<400> 63
   ctaagagcat tctgcagggg ccactgtc 28
<210> 64
   <211> 9
   <212> PRT
   <213> Artificial
<220>
   <223> MAGE1
<400> 64
   Asn Tyr Lys His Cys Phe Pro Glu Ile
   1               5
<210> 65
   <211> 60
   <212> DNA
   <213> Artificial
<220>
   <223> M13-HuVH_201
<400> 65
   gtcctcgcaa caggaaacag ctatgactgt ccactcccag gtgcagctgg tgcagtctgg 60
<210> 66
   <211> 60
   <212> DNA
   <213> Artificial
<220>
   <223> M13-HuVH_202
<400> 66
   gtcctcgcaa caggaaacag ctatgacggt cttgtcccag gtcaccttga aggagtctgg 60
<210> 67
   <211> 60
   <212> DNA
   <213> Artificial
<220>
   <223> M13-HuVH_203
<400> 67
   gtcctcgcaa caggaaacag ctatgactgt ccagtgtgag gtgcagctgg tggagtctgg 60
<210> 68
   <211> 60
   <212> DNA
   <213> Artificial
<220>
   <223> M13-HuVH_204
<400> 68
   gtcctcgcaa caggaaacag ctatgacggt cctgtcccag gtgcagctgc aggagtcggg 60
<210> 69
   <211> 60
   <212> DNA
   <213> Artificial
<220>
   <223> M13-HuVH_206
<400> 69
   gtcctcgcaa caggaaacag ctatgactgt cctgtcacag gtacagctgc agcagtcagg 60
<210> 70
   <211> 59
   <212> DNA
   <213> Artificial
<220>
   <223> M13-HuCH_401
<400> 70
   gtcctcgcaa gtaaaacgac ggccaggagc cagggggaag accgatgggc ccttggtgg 59
<210> 71
   <211> 413
   <212> DNA
   <213> Homo sapiens
<400> 71
   ggtcctgtcc caggtgcagc tgcaggagtc ggggggaggc gtggtccagc ctgggaggtc     60
   cctgagactc tcctgtgcag cctctagatt caccttcagt agctttgcta tgcactgggt    120
   ccgccaggct ccaggcaagg ggctggagtg ggtggcagtt atatcatatg atggaagcaa    180
   taaatactac gcagactccg tgaagggccg attcaccatc tccagagaca attccaagaa    240
   cacgctgtct ctgcaaatga acagcctgag acctgacgac acggctgtgt attactgtgc    300
   gagagccctg cgttattacg atagtagtgg ttatgtggac caatggggcc agggaaccct    360
   ggtcaccgtc tcctcagctt ccaccaaggg cccatcggtc ttccccctgg ctc            413
<210> 72
   <211> 729
   <212> DNA
   <213> Homo sapiens
<400> 72
   cacagcatgg acatgagggt ccccgctcag ctcctggggc tgctaatgct ctggatccct     60
   ggatccagtg cggatgttgt gatgacccag actccactct ctctgtccgt cacccctgga    120
   cagccggcct ccatctcctg caagtctagt cagagcctcc tgcatagtga tgggaagacc    180
   tatttgtttt ggtacctgca gaagccaggc cagtctccac agctcctaat ctatgaagtt    240
   tccaggcggt tctctggagt gccagatagg ttcagtggca gcgggtcagg gacagatttc    300
   acactgagaa tcagccgggt ggaggctgag gatgttgggg tttattactg catgcaaggt    360
   atacacctgt acagttttgg ccaggggacc aagctggaga tcaaacgaac tgtggctgca    420
   ccatctgtct tcatcttccc gccatctgat gagcagttga aatctggaac tgcctctgtt    480
   gtgtgcctgc tgaataactt ctatcccaga gaggccaaag tacagtggaa ggtggataac    540
   gccctccaat cgggtaactc ccaggagagt gtcacagagc aggacagcaa ggacagcacc    600
   tacagcctca gcagcaccct gacgctgagc aaagcagact acgagaaaca caaagtctac    660
   gcctgcgaag tcacccatca ggccctgagc tcgcccgtca caaagagctt caacagggga    720
   gagtgttag                                                            729
<210> 73
   <211> 413
   <212> DNA
   <213> Homo sapiens
<400> 73
   ggtcctgtcc caggtgcagc tgcaggagtc ggggggaggc ctggtcaagc ctggggggtc      60
   cctgagactc tcctgtgcag cctctggatt caccttcagt agctatagca tgaactgggt    120
   ccgccaggct ccagggaagg ggctggagtg ggtctcatcc attagtagta gtagtagtta    180
   catatactac gcagactcag tgaagggccg attcaccatc tccagagaca acgccaagaa    240
   ctcactgtat ctgcaaatga acagcctgag agccgaggac acggctgtgt attactgtgc    300
   gagagaaatt tacgatattt tgactggtta ttcttttgac tactggggcc agggaaccct    360
   ggtcaccgtc tcctcagctt ccaccaaggg cccatcggtc ttccccctgg ctc            413
<210> 74
   <211> 710
   <212> DNA
   <213> Homo sapiens
<400> 74
   cacagcatgg catgagggtc cccgctcagc tcctggggct cctgctactc tggctccgag     60
   gtgccagatg tgacatccag atgacccagt ctccatcctc cctgtctgca tctgtaggag    120
   acagagtcac catcacttgc cgggcaagtc agagcattag cagctattta aattggtatc    180
   agcagaaacc agggaaagcc cctaagctcc tgatctatgc tgcatccagt ttgcaaagtg    240
   gggtcccatc aaggttcagt ggcagtggat ctgggacaga tttcactctc accatcagca    300
   gtctgcaacc tgaagatttt gcaacttact actgtcaaca gagttacagt aggacgttcg    360
   gccaagggac caaggtggaa atcaaacgaa ctgtggctgc accatctgtc ttcatcttcc    420
   cgccatctga tgagcagttg aaatctggaa ctgcctctgt tgtgtgcctg ctgaataact    480
   tctatcccag agaggccaaa gtacagtgga aggtggataa cgccctccaa tcgggtaact    540
   cccaggagag cgtcacagag caggacagca aggacagcac ctacagcctc agcagcaccc    600
   tgacgccgag caaagcagac tacgagaaac acaaactcta cgcctgcgaa gtcacccatc    660
   agggcctgag ctcgcccgtc acaaagagct tcaacagggg agagtgttag               710
<210> 75
   <211> 374
   <212> DNA
   <213> Homo sapiens
<400> 75
   gggaggctcg gtacagcctg gggggtccct gagactctcc tgtgcagcct ctggattcac     60
   ctttagcacc tatgccatga gctgggtccg ccaggctcca gggaaggggc tggagtgggt    120
   ctcaagtttt agtggtaggg gtggtagcac atactacgca gactccgtga agggccggtt    180
   caccatctcc agagacaatt ccaggaacac actgtatctg caaatgaaca gcctgagagc    240
   cgaggacacg gccgtatatt actgtgcgaa ggggacgcta gggctagtgg gagcccctga    300
   ctactggggc cagggaaccc tggtcaccgt ctcctcagct tccaccaagg gcccatcggt    360
   cttcccsctg gctc                                                      374
<210> 76
   <211> 710
   <212> DNA
   <213> Homo sapiens
<400> 76
   ggaaccatga agccccagct cagcttctct tcctcctgct actctggctc ccagatacca      60
   ccggagaaat tgtgttgaca cagtctccag ccaccctgtc tttgtctcca ggggaaagag     120
   ccaccctctc ctgcagggcc agtcagagtg ttagcagcta cttagcctgg taccaacaga     180
   aacctggcca ggctcccagg ctcctcatct atgatgcatc caacagggcc actggcatcc     240
   cagccaggtt cagtggcagt gggtctggga cagacttcat tctcaccatc agcagcctag     300
   agcctgaaga ttttgcactt tattactgtc agcagcgtag caactggcct ctcactttcg     360
   gcggagggac caaggtggag atcaaacgaa ctgtggctgc accatctgtc ttcatcttcc     420
   cgccatctga tgagcagttg aaatctggaa ctgcctctgt tgtgtgcctg ctgaataact     480
   tctatcccag agaggccaaa gtacagtgga aggtggataa cgccctccaa tcgggtaact     540
   cccaggagag tgtcacagag caggacagca aggacagcac ctacagcctc agcagcaccc     600
   tgacgctgag caaagcagac tacgagaaac acaaagtcta cgcctgcgaa gtcacccatc     660
   agggcctgag ctcgcccgtc acaaagagct tcaacagggg agagtgttag                710
<210> 77
   <211> 395
   <212> DNA
   <213> Homo sapiens
<400> 77
   gggaggcctg gtcaagccgg gggggtccct gagactctct tgtgtggcct cgggattcag      60
   attcattaca tatacaatga cttgggtccg tctggctcca gggcgggggc tcgagtgggt     120
   ctcgaccatt ccgagcagtc cgactgacaa tacctacgca gactcagtga agggccgatt     180
   tacgatttct agagacaacg ccaagagctc attgtttctt caaatgagcg gcctgaggga     240
   cgaggatacg gctgtctatt actgtgcgag agtctcaggt gttatggttg aaggtgcagg     300
   aaccgcagga aatgcctttg atctgtgggg tcaggggaca atggtcaccg tctctgcagc     360
   ctccaccaag ggcccatcgg tcttccccct ggctc                                395
<210> 78
   <211> 702
   <212> DNA
   <213> Homo sapiens
<400> 78
   gccaccatgg cctggatccc tctacttctc cccctcctca ttctctgcac agtctctgtg 60
   gcctcctatg agctgacaca gccatcctca gtgtcagtgt ctgcgggaca gacagccagg 120
   atcacctgct caggagatgt tctggcaaaa aaatattctc ggtggttcca gcagaagcca 180
   ggccagaccc ctgtcctact gatttataaa gacactgagc ggccctcagg gatccctgag 240
   cgattctccg gctccagctc agggaccaca gtcaccttga ccatcagcgg ggcccaggtt 300
   gaggatgagg ctgactatta ctgttactct gcggctgaca acacctgggt gttcggcgga 360
   gggaccaagc tgaccgtcct aggtcagccc aaggctgccc cctcggtcac tctgttcccg 420
   ccctcctctg aggagcttca agccaacaag gccacactgg tgtgtctcat aagtgacttc 480
   tacccgggag ccgtgacagt ggcctggaag gcagatagca gccccgtcaa ggcgggagtg 540
   gagaccacca caccctccaa acaaagcaac aacaagtacg cggccagcag ctatctgagc 600
   ctgacgcctg agcagtggaa gtcccacaga agctacagct gccaggtcac gcatgaaggg 660
   agcaccgtgg agaagacagt ggcccctaca gaatgttcat ag 702
<210> 79
   <211> 413
   <212> DNA
   <213> Homo sapiens
<400> 79
   tgtccagtgt gaggtgcagc tggtggagtc tggagctgag gtgaagaagc ctggggcctc 60
   agtgaaggtc tcctgcagga cttctggtta cacctttaag agttatggtg tcagctgggt 120
   gcgacaggcc cctggacaag ggcttgagtg gattggatgg atcagtgttt acaatggtaa 180
   cacacattat gcacagaagt ttcagggcag agtcaccatg aacacagaca catccacgag 240
   cacagcctac ctggacttga ggagcctgag atctgacgac acggccgtct actactgtgc 300
   gagactacgc ggtcagctcg gaataactta cggcatggac gtctggggcc aagggaccac 360
   ggtcaccgtc tcctcagcct ccaccaaggg cccatcggtc ttccccctgg ctc 413
<210> 80
   <211> 726
   <212> DNA
   <213> Homo sapiens
<400> 80
   ctcaccatga ggctccctgc tcagctcctg gggctgctaa tgctctgggt ctctggatcc 60
   agtggggata ttgtgatgac tcagtctcca ctctccctgc ccgtcacccc tggagagccg 120
   gcctccatct cctgcaactc tagtcagagc ctcctacata gtaatgggaa caactatttg     180
   gattggtacc tacagaagcc agggcagtct ccacagctcc tgatctattt gggttctaat     240
   cgggcctccg gggtccctga caggctcagt ggcagtgggt caggcacaga ttttacactg     300
   aaagtcagca aagtggaggc tgaggatgtt ggggtttatt actgcatgca agctctacaa     360
   actccgtgga cttttggccc ggggaccaag ctggagatca aacgaactgt ggctgcacca     420
   tctgtcttca tcttcccgcc atctgatgag cagttgaaat ctggaactgc ctctgttgtg     480
   tgcctgctga ataacttcta tcccagagag gccaaagtac agtggaaggt ggataacgcc     540
   ctccaatcgg gtaactccca ggagagtgtc acagagcagg acagcaagga cagcacctac     600
   agcctcagca gcaccctgac gctgagcaaa gcagactacg agaaacacaa agtctacgcc     660
   tgcgaagtca cccatcaggg cctgagctcg cccgtcacaa agagcttcaa caggggagag     720
   tgttag                                                                726
<210> 81
   <211> 469
   <212> DNA
   <213> Homo sapiens
<400> 81
   gtcctcgcaa caggaaacag ctatgactgt ccactcccag gtgcagctgg tggagtctgg      60
   gggaggcttg gtacagcctg gagggtccct gagactctcc tgtgcagcct ctggattcac     120
   cttcagtagt tatgaaatga actgggtccg ccaggctcca gggaaggggc tggagtgggt     180
   ttcatatatt actagtagtg gtagtaccat atactacgca gactctgtga agggccgatt     240
   caccatctcc agagacaacg ccaagaattc actgtatctg caaatgaaca gcctgagagc     300
   cgaggacacg gctgtttatt actgtgcgag aggggggcgt agggtagtac ccccgggttg       360
   ggcttttgat atccggggcc aagggacaat ggtcaccgtc tcttcagcct ccaccaaggg     420
   cccatcggtc ttccccctgg ctcctggccg tcgttttact tgcgaggac                  469
<210> 82
   <211> 714
   <212> DNA
   <213> Homo sapiens
<400> 82
   gccaccatga cctgctcccc tctcctcctc accctcctca ctcactgtgc agggtcctgg     60
   gcccagtctg tgctgactca gccaccctca gcgtctggga cccccgggca gagggtcacc    120
   atctcttgtt ctggaagcag ctccaacatc ggaagtaata ctgtaaactg gtaccagcag    180
   ctcccaggaa cggcccccaa actcctcatc tatagtaata atcagcggcc ctcaggggtc    240
   cctgaccgat tctctggctc caagtctggc acctcagcct ccctggccat cagtgggctc    300
   cagtctgagg atgaggctga ttattactgt gcagcatggg atgacagcct gaatggtccg    360
   gtgttcggcg gagggaccaa gctgaccgtc ctaggtcagc ccaaggctgc cccctcggtc    420
   actctgttcc cgccctcctc tgaggagctt caagccaaca aggccacact ggtgtgtctc    480
   ataagtgact tctacccggg agccgtgaca gtggcctgga aggcagatag cagccccgtc    540
   aaggcgggag tggagaccac cacaccctcc aaacaaagca acaacaagta cgcggccagc    600
   agctatctga gcctgacgcc tgagcagtgg aagtcccaca gaagctacag ctgccaggtc    660
   acgcatgaag ggagcaccgt ggagaagaca gtggccccta cagaatgttc atag          714
<210> 83
   <211> 421
   <212> DNA
   <213> Homo sapiens
<400> 83
   tgtccactcc caggtgcagc tggtgcagtc tgggggaggc ttagttcaac ctggggggtc     60
   cctgagactc tcctgtgcag cctctggatt cacattcagt agtcattgga tgcactgggt    120
   ccgccaagct ccagggaagg ggctggtgtc cgtctcacgt attaatagtg atgggaagag    180
   cacaacctac gcggactccg tgaagggccg attcaccatc tccagagaca acgccaagaa    240
   cacgctgtat ctgcaaatga acagtctgag agccgacgac acggctgtgt attattgtgc    300
   aagaggaccc gtatatcttg actattgggg ccagggaatt ctggtcaccg tctcctcagc    360
   ctccaccaag ggcccatcgg tcttccccct ggctcctggc cgtcgtttta cttgcgagga    420
   c                                                                    421
<210> 84
   <211> 717
   <212> DNA
   <213> Homo sapiens
<400> 84
   cacagcatgg acatgagggt ccccgctcag ctcctggggc tcctgctgct ctggttccca     60
   ggttccagat gcgacatcca gatgacccag tctccatctt ccgtgtctgc atctgtagga    120
   gacagagtca gcatcacttg tcgggcgagt cagggtattg gcagctggtt agtctggtat    180
   caacaggaac ctgggaaagc ccctaagctc ctgatctatg ctgcctccag tttgcaaagt    240
   ggggtcccat caaggttcag cggcagtgga tctgggacag attttactct caccatcagc    300
   agcctgcaac ctgaagattt tgcaacttac tattgtcaac aggctaacag tttggggctc    360
   actttcggcg gagggaccaa ggtggagatc aaacgaactg tggctgcacc atctgtcttc    420
   atcttcccgc catctgatga gcagttgaaa tctggaactg cctctgttgt gtgcctgctg    480
   aataacttct atcccagaga ggccaaagta cagtggaagg tggataacgc cctccaatcg    540
   ggtaactccc aggagagtgt cacagggcag gacagcaagg acagcaccta cagcctcagc    600
   agcactctgg cgctgagcaa agcagactac gagaaacaca aagtctacgc ctgcgaagtc    660
   acccatcagg gcctgagctc gcccgtcaca aagagcttca acaggggaga gtgttag       717
<210> 85
   <211> 448
   <212> DNA
   <213> Homo sapiens
<400> 85
   tgtccactcc caggtgcagc tggtgcagtc tgggggtgag gtgaagaagc ctggggcctc      60
   agtgaaggtc tcctgtaagg cttctggcta catcttgtca aagtatggca tcagttgggt    120
   gcgacaggcc cctggacaag ggcttgagtg gatggggtgg atcggcattt acagtggaga    180
   cagaaagtac gcacaaaagt tccagggcag agtcaccatg accacagata catccgcggg    240
   catatcctac atggaactga ggagcctgag atctgacgac acggccatgt attactgtgc    300
   gagagccgaa tatagcagca actggtatgg tccgagcgat tcttttgata tctggggcca    360
   aggaacaatg gtcatcgtct ctgcagcttc caccaagggc ccatcggtct tccccctggc     420
   tcctggccgt cgttttactt gcgaggac                                       448
<210> 86
   <211> 713
   <212> DNA
   <213> Homo sapiens
<400> 86
   cacagcatgg acatgaggtc cccgctcagc tcctggggct cctgctgctc tggctcccag     60
   gtgccaaatg tgacatccag atgacccagt ctccttccac cctgtctgca tctgtaggag    120
   acagagtcac catcacttgc cgggccagtc agagtattag tagttggttg gcctggtatc    180
   agcagaaacc agggaaagcc cctaacctcc tgatctataa ggcatctagt ttagaaagtg    240
   gggtcccatc aaggttcagc ggcagtggat ctgggacaga attcactctc accatcagca    300
   gcctgcaggt tgatgatttt gcaacttatt actgccaaca gtataatact taccctactt    360
   ttggcggagg gaccaaggtg gagatcaaac gaactgtggc tgcaccatct gtcttcatct    420
   tcccgccatc tgatgagcag ttgaaatctg gaactgcctc tgttgtgtgc ctgctgaata    480
   acttctatcc cagagaggcc aaagtacagt ggaaggtgga taacgccctc caatcgggta    540
   actcccagga gagtgtcaca gagcaggaca gcaaggacag cacctacagc ctcagcagca    600
   ccctgacgct gagcaaagca gactacgaga aacacaaagt ctacgcctgc gaagtcaccc    660
   atcagggcct gagctcgccc gtcacaaaga gcttcaacag gggagagtgt tag           713

## Description

The present invention relates to a method for analyzing/identifying an antibody gene at one cell level in human B cells, a method for producing an antibody derived from one B cell, and a method for preparing an antibody gene derived from one B cell.

In the body of higher vertebrate animals such as human, there is an acquired immune system for protection of life from foreign bodies including externally invading pathogens such as bacteria and viruses, hazardous substances, and cancer cells. An antibody is a protein, referred to as immunoglobulin, capable of specifically distinguishing among substances such as proteins analogous to one another and is responsible for the antigen-specific humoral immune system in a living body. Since the antibody recognizes numerous foreign bodies, genes encoding a part (the variable region) of antibody undergo rearrangement at the DNA level, resulting in the formation of a population of B lymphocytes having diversified antibody gene sequences. The mechanism of gene diversification through the DNA rearrangement is also found in the sexual reproduction process during which a living organism diversifies genes of its progeny and thereby attempts to make the progeny adapted to environmental changes for survival. The individual single B lymphocyte is known to produce always one type of immunoglobulin encoded by one type of antibody gene.

Conventionally known methods for obtaining an antibody gene include a method for obtaining an antigen-specific antibody gene which involves separating human peripheral blood lymphocytes, removing CD11c-specific cells therefrom using a CD11c-positive antibody and magnetic beads, performing in vitro immunization to induce an antigen-specific human antibody production response, using Epstein-Barr virus to immortalize the peripheral blood lymphocyte cells in which the antigen-specific antibody production response has been induced, isolating antigen-specific B cells, extracting RNA from the antigen-specific antibody-producing B cells, synthesizing cDNA from the extracted RNA, and amplifying an antibody variable region gene by PCR using the synthesized cDNA as a template and primers specific to VH and VL; and an antibody production method which involves contacting a labeled antigen in which the antigen recognized by a desired antibody is labeled, with a cell population containing target cells producing the antibody to cause the binding of labeled antigen to the target cells, separating the resultant labeled target cells, using the separated labeled target cells to prepare an antibody gene possessed thereby, and expressing the prepared antibody gene using an expression vector.

US2007/122852 discloses a method of identifying a B cell comprising contacting a putative B cell containing sample with i) labeled antigen and ii) labeled anti-surface immunoglobulin; isolating B cells; cloning V_{H} and V_{K} genes and incorporating said genes into an expression vector.

Attention has been given in recent years to abnormal clones of immune cells in pathological conditions such as cancer and autoimmune diseases. Given the dynamic adaptation mechanism of immunity, it is easily conceivable that individual cells have different functional characters. From the viewpoint of immune response also, variation in a T-cell receptor or an antibody gene is a phenomenon originally starting from a single cell; functional studies of immune cells are in an era in which studies at one-cell level can no longer be skirted. Particularly, dynamics of individual immune cells under the pathological condition such as cancer have not yet been studied in detail and can be an important study subject in considering a new therapy. The present inventors carried out clinical trials of a dendritic cell vaccine treated with an HLA-A2 or A24 peptide cocktail for cases of metastatic melanoma and have already proposed the analysis and identification of a melanoma peptide-specific antibody gene at one cell level using B cells derived from cancer patients with a confirmed immune response to the cancer-specific antigen peptide (Japanese Patent Application No. 2007-147525). However, this method targeted particular cancer patients in whom a vaccine therapy was established because it was essential in the method to increase B cells producing an antibody to a particular antigen such as a cancer-specific peptide to a certain percentage or more by vaccine administration.

An object of the present invention is to provide a technique for exhaustive analysis of an antibody gene intended for not only samples after vaccine administration but also B cells derived from any cancer-bearing patient, and more specifically to provide a method for analyzing/identifying an antibody gene of one B cell in human and a technique for producing an antibody derived from the one B cell identified, and others.

The present inventors analyzed and identified a melanoma peptide-specific antibody gene at one cell level using immortalized B cells prepared from peripheral blood monocytes derived from melanoma patients before vaccine administration. Specifically, the present inventors have found that the immortalized B cells can be stained with a GST-labeled melanoma-specific cancer antigen MAGE1, an Alexa-labeled anti-GST antibody, and a PE-labeled anti-human IgG antibody and subjected to single cell sorting to separate B cells producing a particular antibody on one cell by one cell basis, and have established a practical technique where a specific antibody gene is efficiently cloned after extracting total RNA from the separated one B cell. In addition, the present inventors considered it important to be able to amplify antibody genes even in normal B cells in view of the diversity of antibody induction, developed a more sensitive technique by improving an RT-PCR technique, and established a practical technique where an antibody gene is efficiently cloned from one B cell using non-immortalized B cells, thereby accomplishing the present invention.

Thus, the present invention relates to:
[1] A method for analyzing/identifying an antibody gene in one B cell derived from a human, successively comprising the steps of (A), (B), (C), (D), (E), (F) and (G), wherein the human is a cancer-bearing patient in whom a vaccine therapy has not been established, wherein the vaccine therapy increases B cells producing an antibody to a cancer-specific peptide to a certain percentage or more:
   (A) harvesting peripheral blood mononuclear cells from peripheral blood obtained from the human;
   (B) producing an immortalized B cell (EBV-B cell) line from the obtained peripheral blood mononuclear cells using Epstein-Barr virus (EBV);
   (C) labeling the EBV-B cells with a marker-labeled antigen and with an antibody which is capable of recognizing a human antibody and is labeled with a marker different from the marker, wherein the antigen is a cancer-specific antigen peptide or cancer-specific antigen protein for a cancer of the cancer-bearing patient;
   (D) separating EBV-B cells, that express an antibody recognizing the antigen on the cell membrane, on one cell by one cell basis;
   (E) extracting total RNA from the one cell and synthesizing cDNA by reverse transcription reaction;
   (F) using the synthesized cDNA as a template to perform a PCR reaction using a pair of primers specific for a human antibody heavy chain region gene, a PCR reaction using a pair of primers specific for a human antibody light chain κ region gene, or a PCR reaction using a pair of primers specific for a human antibody light chain λ region gene to amplify each of the region gene fragments; and
   (G) analyzing/determining the base sequence of the amplified gene fragment.
[2] The analyzing/identifying method according to [1], wherein the cancer-specific antigen peptide or cancer-specific antigen protein is MAGE1, MAGE2, MAGE3, MART1, tyrosinase, or gp100.
[3] A method for preparing an antibody gene of one B cell derived from a human, successively comprising the steps of (a), (b), (c), (d), (c) and (f), wherein the human is a cancer-bearing patient in whom a vaccine therapy has not been established, wherein the vaccine therapy increases B cells producing an antibody to a cancer-specific peptide to a certain percentage or more:
   (a) harvesting peripheral blood mononuclear cells from peripheral blood obtained from the human;
   (b) producing an immortalized B cell (EBV-B cell) line from the obtained peripheral blood mononuclear cells using Epstein-Barr virus (EBV);
   (c) labeling the EBV-B cells with a marker-labeled antigen and with an antibody which is capable of recognizing a human antibody and is labeled with a marker different from the marker, wherein the antigen is a cancer-specific antigen peptide or cancer-specific antigen protein for a cancer of the cancer-bearing patient;
   (d) separating EBV-B cells, that express an antibody recognizing the antigen on the cell membrane, on one cell by one cell basis;
   (e) extracting total RNA from the one cell and synthesizing cDNA by reverse transcription reaction; and
   (f) using the synthesized cDNA as a template to perform a PCR reaction using a pair of primers specific for a human antibody heavy chain region gene, a PCR reaction using a pair of primers specific for a human antibody light chain κ region gene, or a PCR reaction using a pair of primers specific for a human antibody light chain λ region gene to amplify each of the region gene fragments.
[4] The method for preparing an antibody gene according to [3], wherein the cancer-specific antigen peptide or cancer-specific antigen protein is MAGE1, MAGE2, MAGE3, MART1, tyrosinase, or gp100.
[5] A method for producing an antibody of one B cell derived from a human, comprising preparing an antibody gene according to the method of [3] or [4] and expressing the antibody gene using an expression vector.

According to the present invention, genes of functional antibodies can be analyzed and identified for human B cells at one B cell level and genes of antibodies to specific immune epitopes and tumor antigens can be exhaustively analyzed and identified; thus, the present invention is extremely useful for the screening of a new cancer treatment target, the development of a cancer therapeutic agent, and further for the future tailor-made medicine and diagnosis for cancer.
[Figure 1] A drawing showing the schematic of the method for detecting EBV-B cells expressing an IgG antibody specific for a GST-labeled antigen according to the present invention.
[Figure 2] A drawing showing incorporation into the donor plasmid for preparing baculovirus, containing a GFP-MAGE1 gene sequence, used in the present invention.
[Figure 3] A drawing showing the schematic of the single-cell RT-PCR cloning according to the present invention.
[Figure 4] A drawing showing the method for preparing a recombinant antibody according to the present invention.
[Figure 5] A drawing showing the design of the control RNA template for real-time PCR analysis, used in the present invention.
[Figure 6] A drawing showing a method for preparing the control RNA template for real-time PCR analysis, used in the present invention.
[Figure 7] A drawing showing the experimental procedure for a kit for real-time PCR (TaqMan Gene Expression Cell-to-Ct kit), used in the present invention.
[Figure 8] A drawing showing the results of flow cytometry analysis according to the present invention. The analysis results of EBV-B cells derived from a patient before administration (MEL-018Pre) and after 6 times administration of a dendritic cell vaccine (MEL-018Post) in case MEL-018 are shown.
[Figure 9A] A drawing showing the results of flow cytometry analysis according to the present invention. The analysis results of EBV-B cells derived from cases MEL-016, MEL-017, MEL-018 and MEL-021 are shown.
[Figure 9B] A drawing showing the results of flow cytometry analysis according to the present invention. The analysis results of EBV-B cells derived from cases MEL-022, MEL-023, MEL-SCC004 and MEL-SCC005 are shown.
[Figure 9C] A drawing showing the results of flow cytometry analysis according to the present invention. The analysis results of EBV-B cells derived from cases MEL-001post, MEL-006post, MEL-018post and MEL-014* are shown.
[Figure 10] A drawing showing the results of examining the expression of an anti-MAGE-1 antibody in an EBV-B cell of the present invention by immunohistochemical staining.
[Figure 11] A drawing showing the results of the purification (metal chelate affinity purification) of an scF antibody according to the present invention.
[Figure 12] A drawing showing the results of the purification (anion exchange purification) of an scF antibody according to the present invention.
[Figure 13] A drawing showing the results of the analysis of an scF antibody according to the present invention by western blotting.
[Figure 14] A drawing showing a calibration curve in the real-time PCR for examining the expression amount of a gene in an EBV-B cell according to the present invention.
[Figure 15] A drawing showing the results of examining the expression amount (the number of copies) of β-actin gene in an EBV-B cell by the real-time PCR according to the present invention.
[Figure 16] A drawing showing the results of examining the expression amount (the number of copies) of IgG gene in the EBV-B cell by the real-time PCR according to the present invention.
[Figure 17] A drawing showing the results of measuring the antibody titer of an anti-CMV-pp65 antibody in a human serum.
[Figure 18] A drawing showing the results of staining B cells with a GST-labeled CMVpp65 antigen protein, an Alexa488-labeled anti-GST antibody and a PE-labeled anti-human IgG antibody.
[Figure 19] A drawing showing the results of staining B cells with CMVpp65 protein.
[Figure 20] A drawing showing the results of performing the stain identification and capture of CVMpp65 antigen-positive B cells using cell microarray.
[Figure 21] A drawing showing the results of detecting calcium ion influx using CMV-pp65-positive wells.
[Figure 22] A drawing showing the procedure of the single-cell RT-PCR method for cloning an IgG gene according to the present invention.
[Figure 23] A drawing showing the results of comparing the efficiency of the single-cell RT-PCR method for cloning an IgG gene according to the present invention.
[Figure 24] A drawing showing the procedure of the single-cell RT-PCR method for cloning an IgG gene according to the present invention.
[Figure 25] A drawing showing the results of the single-cell RT-PCR method for cloning an IgG gene according to the present invention.
[Figure 26] A drawing showing the number of B cells in which an IgG gene was successfully cloned by the single-cell RT-PCR method for cloning the gene according to the present invention.
[Figure 27] A drawing showing the results of repertoire analysis of antibody genes successfully cloned by the method of the present invention.
[Figure 28] A drawing showing the primer sequences for 2^{nd} PCR (SEQ ID NOS: 65 to 70) used in the present invention.
[Figure 29] A drawing showing the base sequence of the antibody heavy chain gene (#081215-1) (SEQ ID NO: 71) obtained by the method of the present invention.
[Figure 30] A drawing showing the base sequence of the antibody light chain κ gene (#081215-1) (SEQ ID NO: 72) obtained by the method of the present invention.
[Figure 31] A drawing showing the base sequence of the antibody heavy chain gene (#081215-5) (SEQ ID NO: 73) obtained by the method of the present invention.
[Figure 32] A drawing showing the base sequence of the antibody light chain κ gene (#081215-5) (SEQ ID NO: 74) obtained by the method of the present invention.
[Figure 33] A drawing showing the base sequence of the antibody heavy chain gene (#081215-19) (SEQ ID NO: 75) obtained by the method of the present invention.
[Figure 34] A drawing showing the base sequence of the antibody light chain κ gene (#081215-19) (SEQ ID NO: 76) obtained by the method of the present invention.
[Figure 35] A drawing showing the base sequence of the antibody heavy chain gene (#081215-23) (SEQ ID NO: 77) obtained by the method of the present invention.
[Figure 36] A drawing showing the base sequence of the antibody light chain λ gene (#081215-23) (SEQ ID NO: 78) obtained by the method of the present invention.
[Figure 37] A drawing showing the base sequence of the antibody heavy chain gene (#090204-15) (SEQ ID NO: 79) obtained by the method of the present invention.
[Figure 38] A drawing showing the base sequence of the antibody light chain κ gene (#090204-15) (SEQ ID NO: 80) obtained by the method of the present invention.
[Figure 39] A drawing showing the base sequence of the antibody heavy chain gene (#090219-11) (SEQ ID NO: 81) obtained by the method of the present invention.
[Figure 40] A drawing showing the base sequence of the antibody light chain λ gene (#090219-11) (SEQ ID NO: 82) obtained by the method of the present invention.
[Figure 41] A drawing showing the base sequence of the antibody heavy chain gene (#090225-100) (SEQ ID NO: 83) obtained by the method of the present invention.
[Figure 42] A drawing showing the base sequence of the antibody light chain κ gene (#090225-100) (SEQ ID NO: 84) obtained by the method of the present invention.
[Figure 43] A drawing showing the base sequence of the antibody heavy chain gene (#090225-104) (SEQ ID NO: 85) obtained by the method of the present invention.
[Figure 44] A drawing showing the base sequence of the antibody light chain κ gene (#090225-104) (SEQ ID NO: 86) obtained by the method of the present invention.

In the present invention the human from which the peripheral blood is harvested in the step (A) is a cancer patient being in a cancer-bearing state. The cancer patient is an unvaccinated cancer patient. The cancer may be a solid cancer or a blood cancer. Here, the solid cancer includes a sarcoma and a carcinoma; specific examples include melanoma, fibrosarcoma, mucosal sarcoma, liposarcoma, chondrosarcoma, osteogenic sarcoma, chordoma, angiosarcoma, lymphangiosarcoma, lymphangioendotheliosarcoma, synovioma, mesoepithelioma, Ewing tumor, leiomyosarcoma, rhabdomyosarcoma, stomach cancer, esophageal cancer, large bowel cancer, colon cancer, rectal cancer, pancreas cancer, breast cancer, ovarian cancer, prostatic cancer, squamous cell carcinoma, basal cell cancer, adenocarcinoma, sweat gland carcinoma, sebaceous carcinoma, papillary cancer, papillary adenocarcinoma, cystadenocarcinoma, bone marrow cancer, bronchogenic cancer, renal cell cancer, ureter cancer, liver cancer, bile duct cancer, choriocarcinoma, seminoma, embryonal carcinoma, Wilms' tumor, cervical cancer, endometrial cancer, testicular cancer, small-cell lung cancer, non-small-cell lung cancer, bladder cancer, epithelial cancer, neuroglioma, astrocytoma, myeloblastoma, craniopharyngeal cancer, laryngeal cancer, tongue cancer, astroependymoma, pinealoma, angioblastoma, acoustic nerve tumor, oligodendroglioma, meningioma, peritoneal dissemination, teratoma, neuroblastoma, medulloblastoma, and retinoblastoma. The above blood cancer includes a myeloma and a lymphoma; specific examples thereof can include acute myelocytic leukemia, acute myelogenous leukemia, chronic myelocytic leukemia, acute lymphatic leukemia, chronic lymphatic leukemia, Hodgkin disease, non-Hodgkin disease, adult T cell leukemia, and multiple myeloma.

To produce an immortalized B cell (EBV-B cell) line from peripheral blood mononuclear cells using Epstein-Barr virus (EBV) in the above step (B), the peripheral blood mononuclear cells may be cultured with EBV in the presence of feeder cells to immortalize the peripheral blood mononuclear cells; a labeled antigen peptide may also be used in addition to an anti-CD19 antibody and an anti-human IgG antibody, markers for B lymphocytes, to identify the presence of a desired antigen-specific antibody-producing EBV-B cell line.

The antigen in the above steps (C) an (c) is not particularly limited provided that it is a cancer-specific antigen peptide or protein for a cancer of the cancer patient. Among others, preferred examples thereof can include peptides or proteins highly expressed specifically in cancer cells (cancer-specific peptides or cancer-specific proteins). More specifically, preferred examples thereof can include cancer-specific peptides and cancer-specific proteins such as MAGE1, MAGE2, MAGE3, MART1, tyrosinase, and gp100. The antigen may be variously modified provided that it has a function as an antigen; for example, it may be a so-called fused protein in which another peptide or protein moiety is added to a functional portion as antigen (an epitope) or in which a sugar chain or an aliphatic chain is added. Examples of the marker in the above step (C) can include fluorescent substances such as Alexa Fluor 488, green fluorescent protein (GFP), fluorescein isothiocyanate (FITC), phycoerythrin (PE), and tetramethylrhodamine isothiocyanate (TRITC), chemiluminescent substances such as luminol, isoluminol, and acridinium derivatives, biotin, and magnet beads. In addition, the above step (C) may be a step of labeling EBV-B cells with a marker-labeled antigen, an antibody to the marker, and an antibody which is capable of recognizing a human antibody and is labeled with a marker different from the above marker; examples of the marker here can include epitope tags such as glutathione S-transferase (GST), c-Myc, HA, and FLAG. An antibody specifically recognizing any of these epitope tags can be used as an antibody to a marker. The antibody specifically recognizing any of these epitope tags may use one labeled with a marker such as the above fluorescent substance and chemiluminescent substance. These labeling can be carried out by a conventional method. See, for example, Molecular Cloning, Third Edition, Cold Spring Harbor Laboratory Press, New York. Preferred examples of the antibody capable of recognizing a human antibody in the step (C) can include an anti-human anti-human IgG antibody. In the step (C), for example, the labeling is preferably carried out under conditions not causing the dropping off of a cell membrane-bound antibody of cancer antigen-specific antibody-producing B cells, such as using GST-labeled cancer specific antigen protein as a marker-labeled cancer-specific antigen protein, an Alexa-labeled anti-GST antibody, and a PE-labeled anti-human anti-human IgG antibody as a different marker-labeled antibody capable of recognizing a human antibody.

In the above steps (D) and (d), EBV-B cells expressing an antibody recognizing the antigen are separated on one cell by one cell basis. To separate B cells on one cell by one cell basis, a suitable technique is used depending on the type of the marker employed. For example, when a fluorescent substance is used as a marker, B cells are preferably separated on one cell by one cell basis by flow cytometry (single cell sorter) using fluorescence as an indicator. Flow cytometry can efficiently separate cells with high accuracy. The adoption of biotin as a marker also enables the separation thereof on one cell by one cell basis using a binding reaction with avidin. Similarly, when magnet beads are used, good separation is also possible using the magnet. Separation may also be performed on one cell by one cell basis using cell microarray, a micromanipulator, or a micro mesh filter.

In the above steps (E) and (e), total RNA is extracted from one antibody-producing B cell separated on one cell by one cell basis and cDNA are synthesized by reverse transcription reaction. The separation of total RNA, the separation and purification of mRNA, and the obtaining, cloning of cDNA and the like may be all carried out according to ordinary methods (see, for example, Molecular Cloning: A laboratory Mannual, 2nd Ed., Cold Spring Harbor Laboratory, Cold Spring Harbor, NY.,1989).

In the above steps (F) and (f), the synthesized cDNA are used as a template to perform a PCR reaction using a pair of primers specific for a human antibody heavy chain region gene, a PCR reaction using a pair of primers specific for a human antibody light chain κ region gene, or a PCR reaction using a pair of primers specific for a human antibody light chain λ region gene to amplify each of the region gene fragments. The pair of primers specific for a human antibody heavy chain region gene, a human antibody light chain κ region gene or a human antibody light chain λ region gene is not particularly limited provided that it is a pair of primers specific for the sequence of each region gene. However, examples of the pair of primers specific for a human antibody heavy chain region gene can include a pair of primers consisting of one or two or more sequences of the base sequences represented by SEQ ID NOS: 1 to 24 and the base sequence represented by SEQ ID NO: 25 or 26; examples of the pair of primers specific for a human antibody light chain κ region gene can include a pair of primers consisting of one or two or more sequences of the base sequences represented by SEQ ID NOS: 27 to 37 and the base sequence represented by SEQ ID NO: 38; and examples of the pair of primers specific for a human antibody light chain λ region gene can include a pair of primers consisting of one or two or more sequences of the base sequences represented by SEQ ID NOS: 39 to 61 and one or two sequences of the base sequences represented by SEQ ID NOS: 62 and 63. The base sequences of the amplified gene fragments are analyzed and determined by an ordinary method in the above step (G). Examples of the type of the antibody can include IgA, IgD, IgE, and IgM in addition to IgG.

The gene fragments amplified in the steps (F) and (f) can be used to prepare an antibody gene derived from one B cell. Specifically, a human antibody heavy chain gene can be prepared by PCR reaction using a pair of primers specific for a human antibody heavy chain region gene. A human antibody light chain gene can also be prepared by PCR reaction using a pair of primers specific for a human antibody light chain κ region gene or PCR reaction using a pair of primers specific for a human IgG light chain λ region gene. In addition, a human antibody heavy chain variable region gene fragment can be prepared by PCR reaction using a pair of primers specific for the human antibody heavy chain variable region gene fragment. A human light chain variable region gene fragment can be prepared by PCR reaction using a pair of primers specific for a human antibody light chain κ variable region gene fragment or PCR reaction using a pair of primers specific for a human IgG light chain λ variable region gene fragment. These prepared human antibody genes can also be subcloned for amplification. However, when a genomic DNA is used as a template, their effective amplification cannot be expected because exons of are separated.

The use of the following method of the present inventors (Japanese Patent Application No. 2007-92968) enables the efficient and abundant production of a human ScFv fragment. The human antibody heavy chain variable region gene fragment (heavy chain fragment) and the human light chain variable region gene fragment (light chain fragment) are amplified by the PCR method. These heavy chain and light chain fragments are amplified by the PCR method in the forms of a heavy chain conjugate fragment containing the heavy chain fragment-heavy chain linker sequence-restriction enzyme XbaI recognition sequence and a light chain conjugate fragment containing a restriction enzyme NheI recognition sequence-light chain linker sequence-light chain fragment, respectively. The heavy chain conjugate fragment and the light chain conjugate fragment are digested with the restriction enzyme XbaI and the restriction enzyme NheI, respectively and then linked by ligation. The ligation product is digested with the restriction enzyme XbaI and the restriction enzyme NheI and then amplified by the PCR method in the form of a human single-chain antibody gene (ScFv) fragment consisting of a heavy chain fragment-linker sequence-light chain fragment.

In the steps (H) and (h), the human antibody heavy chain gene and the human antibody light chain gene, which are the gene fragments amplified in the steps (F) and (f), can be expressed using an expression vector to produce an antibody derived from the one B cell. The expression vector is not particularly limited provided that it is suitable for the expression of an antibody gene; however, examples thereof can include an SV40 virus vector, an EB virus vector, and a papilloma virus vector in addition to an adenovirus vector used for transient expression in all cells (except hematocytic cells) including non-dividing cells (Science, 252, 431-434, 1991), a retroviral vector used for long-term expression in dividing cells (Microbiology and Immunology, 158, 1-23, 1992), and an adeno-associated virus vector also capable of being introduced into non-pathogenic and non-dividing cells and used for long-term expression (Curr. Top. Microbiol. Immunol., 158, 97-129, 1992). To increase the expression efficiency, a selection marker gene may also be introduced into these virus vectors in addition to control sequences such as a promoter sequence and an enhancer sequence. The introduction of the antibody genes into the expression vector can be carried out by a well-known method using restriction enzymes and a DNA ligase (see, e.g., Molecular Cloning, Third Edition, 1.84, Cold Spring Harbor Laboratory Press, New York).

Preferably, the human antibody heavy chain gene and the human antibody light chain gene are typically inserted into separate expression vectors and a host is co-transformed with these two recombinant vectors to express the heavy chain and the light chain in the same cells. The host is not particularly limited provided that it can be transformed with the recombinant vectors to hold the introduced antibody genes in a state capable of expression; examples thereof can include Vero cells, Hela cells, CHO cells, WI38 cells, BHK cells, COS-7 cells, and MDCK cells. Methods for transforming the host with the recombinant vector can include, for example, a ripofectin method, an electroporation method, and a calcium phosphate method. In this manner, a monoclonal antibody can be produced from one B cell without using a hybridoma. Escherichia coli can also be transformed with a phagemid vector or phage vector having the human ScFv fragment incorporated to produce a phage-displayed human single stranded antibody using the transformed Escherichia coli.

An antibody gene of a cancer patient can be analyzed and identified by the analyzing/identifying method for an antibody gene derived from one B cell according to the present invention to provide information on an overview of types of cancer antigen-specific antibodies produced in the body of an individual patient. In addition, the method for producing an antibody derived from one B cell according to the present invention can provide a cancer antigen-specific antibody in large quantities, enabling the tailor-made diagnosis and treatment of an individual patient. The antibody gene obtained by the method for preparing an antibody gene derived from one B cell according to the present invention is advantageously used in producing a cancer antigen-specific antibody in large quantities and also utilized for the tailor-made diagnosis of an individual patient. Particularly, the method of the present invention has a large advantage of being capable of providing a human antibody not in the form of a partial human antibody as conventionally obtained by immunizing mice and producing a hybridoma but in the form of a 100% human antibody formed by actual amplification in the human body.

The present invention will be described more specifically below with reference to Examples. However, these Examples are not intended to limit the technical scope of the present invention.

### Example 1

### [Harvesting of Peripheral Blood Mononuclear Cells and Production of Immortalized B Cell Line]

Twelve samples of peripheral blood mononuclear cells (PBMC) were harvested from peripheral bloods derived from melanoma patients before and/or after dendritic cell vaccine administration (including the same patient before and after the vaccine administration). The dendritic cell vaccine was one treated with 5 types of peptide: HLA-A24 restriction MAGE1₁₃₅₋₁₄₃ (the amino acid sequence of MAGE1₁₃₅₋₁₄₃ is shown in SEQ ID NO: 64), MAGE2, MAGE3, gp100 and tyrosinase or 5 types of peptide: HLA-A2 restriction MAGE2, MAGE3, gp100, MART1 and tyrosinase.

### [Production of Immortalized B Cell Line]

Using Epstein-Barr virus (EBV), an immortalized B cell line (an EBV-transformed B cell line; hereinafter referred to as an EBV-B cell line) was produced from the harvested PBMC. Specifically, a human fibroblast cell line (MRC-5; ATCC cat. CCL-171) as a feeder was propagated to 90% confluency in a 25-cm² flask (medium: MEM + 10% FBS) and then subjected to 30 to 40 Gy irradiation. Twenty-four hours after, PBMC harvested in Example 1 were suspended in a medium (IMDM + 20% FBS) to 1 to 2 × 10⁷ cells/4 ml, which was then added to the above culture MRC-5. Thereafter, 1 ml of an EBV solution (EBV strain B95-8, ATCC cat. VR-1492) was added to a 25-ml flask and cultured under gas conditions of 37°C and 5% CO₂. The medium was exchanged 48 hours after the start of culture and then exchanged every 4 days. The propagation of B cells was identified after 3 to 4 weeks, and B cells immortalized by EBV (EBV-B cells) were recovered to prepare 12 samples of EBV-B cells in total (MEL-001post, MEL-006post, MEL-014, MEL-016, MEL-017, MEL-018, MEL-018post, MEL-021, MEL-022, MEL-023, MEL-SCC004, and MEL-SCC005). Of the 12 cell lines prepared, MEL-014, MEL-016, MEL-017, MEL-018, MEL-021, MEL-022, MEL-023, MEL-SCC004, and MEL-SCC005 are derived from patients before vaccine administration and MEL-001post, MEL-006post, and MEL-018post are derived from patients after vaccine administration. MEL-018 and MEL-018post are EBV-B cells produced from PBMC before vaccine administration (MEL-018) and after vaccine administration (MEL-018post) derived from the same patient.

### Example 2

### [Analysis of EB-B cells by Staining and Flow Cytometry]

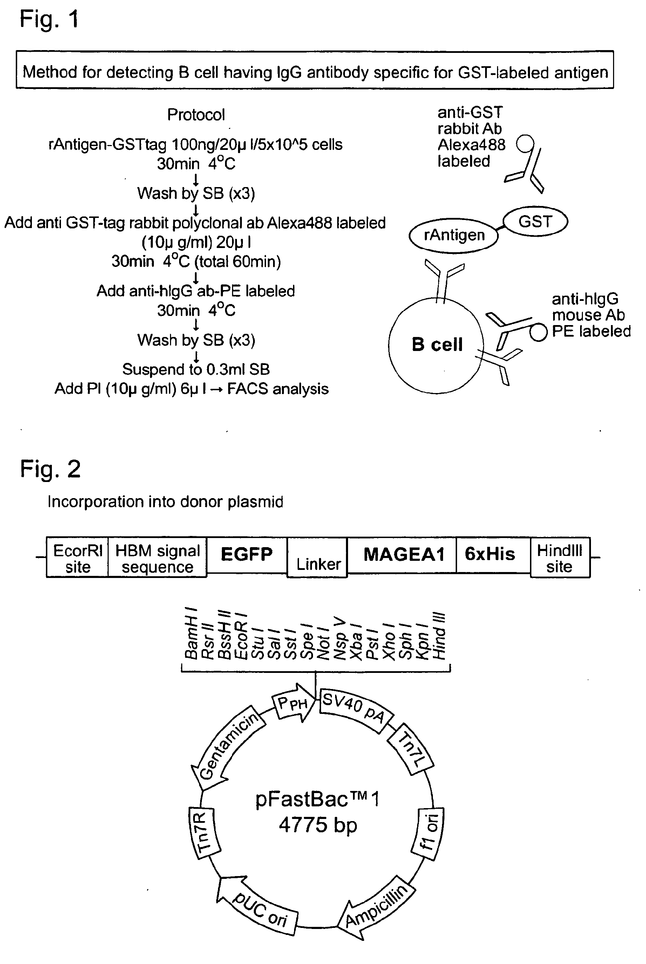

EB-B cells were stained with a GST-labeled melanoma-associated recombinant protein, an Alexa Fluor 488-labeled anti-GST antibody, and a PE-labeled anti-human IgG antibody and analyzed by flow cytometry. The schematic of this experiment is shown in Figure 1. Of the 6 GST-labeled melanoma-associated recombinant proteins, MAGE1, MAGE2, MAGE3, MART1, and tyrosinase were purchased from Abnoba. and gp100, from Abcam. GST protein as a negative control was synthesized using Escherichia coli. The Alexa Fluor 488-labeled anti-GST polyclonal antibody (hereinafter referred to as Alexa-anti-GST antibody) was purchased from Invitrogen and the PE-labeled anti-human IgG antibody (hereinafter referred to as PE-anti-hIgG), from BD Phaimingen.

The staining was carried out by the following procedure. First, EBV-B cells were washed with a sorter buffer (PBS + 2% FBS + 0.1% NaN3) and then adjusted to an amount of 20 µl/tube. A solution of GST or each GST-labeled protein adjusted to a concentration of 100 ng/20 µl with PBS (0.2% BSA) was added to the EBV-B cells, which was then reacted at 4°C for 30 minutes. Next, 20 µl (10 µg/ml) of Alexa-anti-GST antibody was added thereto, which was then reacted at 4°C for 30 minutes, followed by adding 20 µl of PE-hIgG antibody for reaction at 4°C for 30 minutes. The stained EBV-B cells were analyzed by a flow cytometer (FACS-CANTO, BD). For identification of live cells, PI staining was performed immediately before measurement.

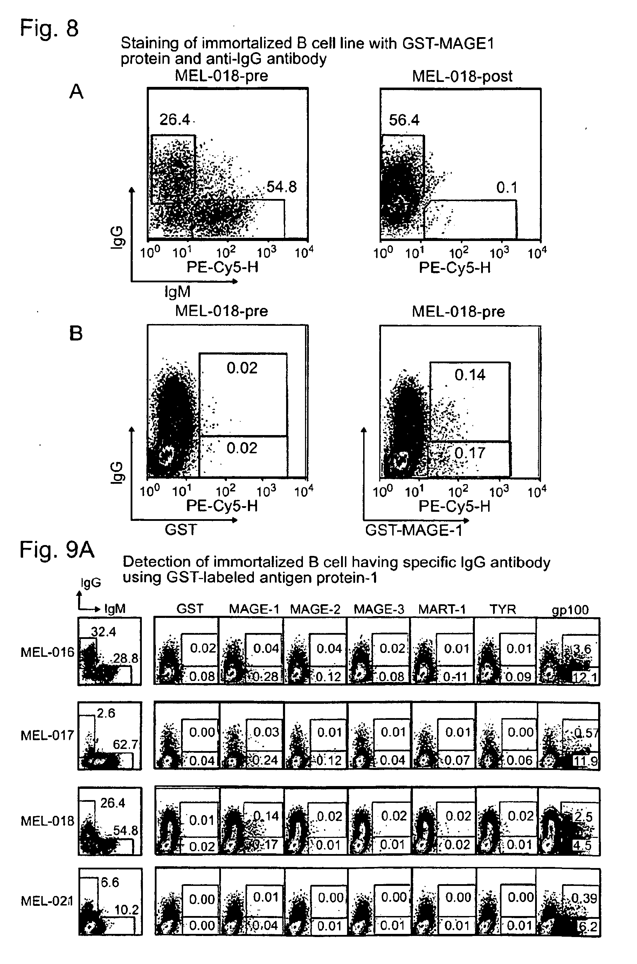

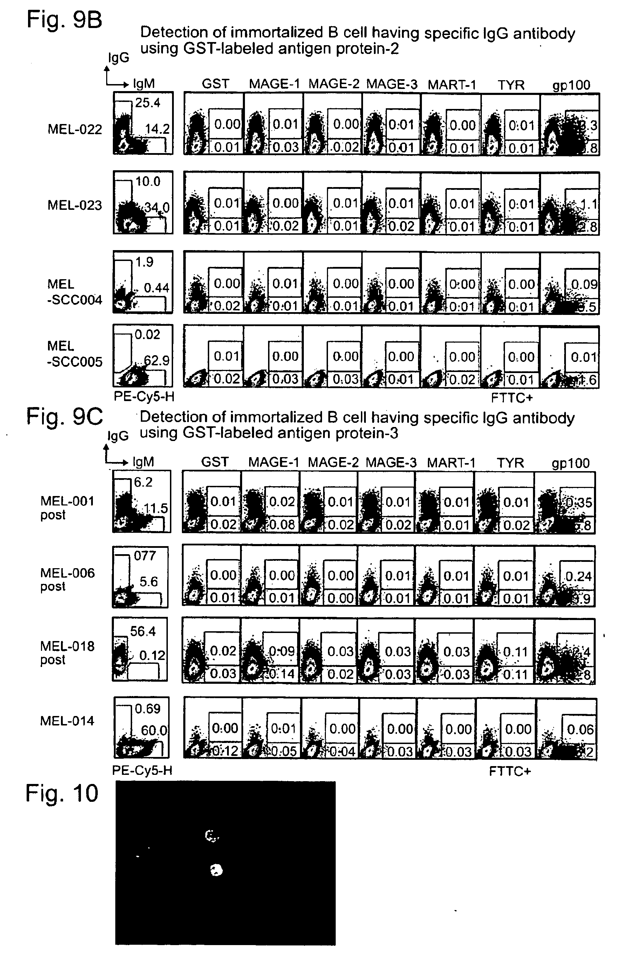

The analysis results of flow cytometry are shown in Figures 8 and 9. Figure 8 shows data with MAGE1, before administration (MEL-018Pre) and after 6 times dendritic cell vaccine administration (MEL-018Post) in case MEL-018. Figure 8A shows an evident increase in the proportion of IgG antibody positivity in IgG and IgM fractions of EBV-B cell line. In addition, as shown in Figure 8B, the proportion of IgG⁺/MAGE1⁺ cell population was increased to 0.14% in staining with GST-MAGE1 (0.02% for only GST as a negative control), and an evident increase in B cells having An IgG antibody to MAGE1 was detected. Figures 9A to 9C show analysis data with other cancer-specific antigen proteins. The proportion of positive cases of an IgG antibody to MAGE1 was 5/12 (0.03 to 0.14%) in staining with these melanoma antigen propteins (fused to GST) . In addition, IgM and IgG antibodies to gp100 were detected in all cases (gp100⁺/IgG⁺; 0.06 to 3.6%). Interestingly, both IgM and IgG antibodies to tyrosinase were detected in MEL-018Post (after dendritic cell vaccine administration).

### Example 3

### [Immunohistochemical Staining of EBV-B Cell]

Green fluorescence protein (GFP)-labeled MAGE-1 protein (hereinafter referred to as GFP-MAGE1) was synthesized and used to perform the immunohistochemical staining of EBV-B cells. GFP-MAGE1 was made using a production system of baculovirus. As shown in Figure 2, a gene sequence encoding GFP-MAGE1 was incorporated into a donor plasmid (pFastBac) and prepared in the form of a Bacmid DNA in Escherichia coli. The Bacmid DNA was transfected into insect-derived cells Sf9, and baculovirus produced in a culture supernatant was recovered. High-Five cells were infected with high titer baculovirus containing a GFP-MAGE1 gene sequence and subjected to shaking (72 rpm) culture at 27°C for 50 to 64 hours. The cultured cells were recovered, lysed by freezing and thawing, and centrifuged to recover a supernatant. GFP-MAGE1 was purified from the recovered supernatant using a metal chelate affinity gel utilizing His-tag and desalted with PD-10 column, and then the protein was concentrated using an ultrafiltration column. GFP-MAGE1 was stored at 4°C until use. When used in the experiment, the stored GFP-MAGE1 (4 mg/ml) was subjected to the addition of an equal volume of 2-mercaptoethanol (20 mM) at the time of use, which was then reacted at 4°C overnight and then adjusted to a concentration of 100 µg/ml using a sorter buffer (final concentration 20 µg/ml, reacted at 4°C for one hour). This GFP-MAGE1 was used to perform the immunohistochemical staining of EBV-B cells produced in Example 1, and cells to which GFP-MAGE1 bound were detected under a fluorescence microscope. As a result of staining MEL-018Pre-derived EBV-B cells, the whole bodies were lightly stained; however, the percentage of stained cells was considerably low (about 2% of IgG⁺ B cells). As shown in Figure 10, the presence of B cells positively stained in a patched form and believed to express an anti-MAGE1 antibody was identified in a high-power field (× 200).

### Example 4

### [Single Cell Sorting of EBV-B Cells]

Single cell sorting was carried out for the purpose of separating GFP-MAGE1⁺/PE-anti-hIgG⁺ EBV-B cells on one cell by one cell basis. For sorting, BD FACSAria^{™} cell sorter (from BD Science) equipped with a module for single cell sorting was used. For sorting, cells were separated into a 96-well plate (MicroAmp (R) Optical 96-well Reaction Plate; from Applied Biosystem) on one cell by one cell basis using 100 µm nozzle and the conditions of sort setup: low, flow rate: 5,000 events/sec and drop delay: 25.73.

### Example 5

### [Single Cell RT-PCR Cloning]

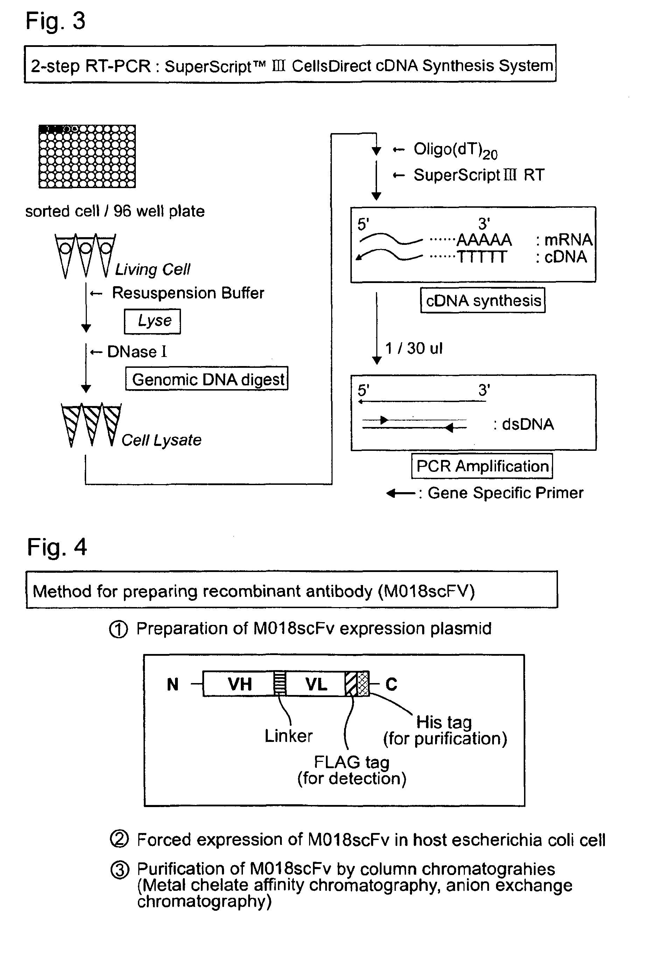

The EBV-B cells separated on one cell by one cell basis in Example 4 were subjected to single cell RT-PCR cloning. The experimental outline of cloning is shown in Figure 3. Primers for PCR, specific for regions of the human IgG heavy chain gene, the human IgG light chain κ gene, or the human IgG light chain λ gene were designed for cloning primers. Primer base sequences specific for the human IgG heavy chain gene were shown in SEQ ID NOS: 1 to 26 (SEQ ID NOS: 1 to 24: forward primers, SEQ ID NOS: 25 and 26: reverse primers) ; primer base sequences specific for the human IgG light chain κ gene, in SEQ ID NOS: 27 to 38 (SEQ ID NOS: 27 to 37: forward primers, SEQ ID NO: 38: reverse primers) ; and primer base sequences specific for the human IgG light chain λ gene, in SEQ ID NOS: 39 to 63 (SEQ ID NOS: 39 to 61: forward primers, SEQ ID NOS: 62 and 63: reverse primers). The size of the gene fragments amplified with primer mixes was about 1,400 bp for the human IgG heavy chain gene, about 700 bp for the human IgG light chain κ gene, and about 700 bp for the human IgG light chain λ gene.

First, RNA was extracted from the single cell separated, and cDNA was synthesized by reverse transcription reaction. Super Script^{™} III Cells Direct cDNA Synthesis System (cat. 18080-300, from Invitrogen) was used for synthesis thereof. A resuspension buffer (10 µl) and a lysis enhancer solution (1 µl) were added thereto, which was then treated at 75°C for 10 minutes using a thermal cycler. Subsequently, 5 µl of DNase I (1 U/µl) and 1.6 µl of 10 × DNase I buffer were added thereto, which was then mixed by pipetting and incubated at room temperature for 5 minutes. The plate was slightly centrifuged, to which 1.2 µl of 25 mM EDTA was added, followed by incubation at 70°C for 5 minutes using a thermal cycler. The plate was then slightly centrifuged, and 2 µl of 50 mM Oligo (dT)₂₀ and 1 µl of 10 mM dNTP mix were added to each well on ice. After mixing by pipetting, the plate was treated at 70°C for 5 minutes using a thermal cycler and incubated for 2 minutes on ice. The plate was slightly centrifuged, to which 6 µl of 5 × RT buffer, 1 µl of RNase OUT^{™} (40 U/µl), 1 µl of Super Script^{™} III RT (200 U/µl), and 1 µl of 0.1 M DTT were then added again on ice, followed by mixing by pipetting. The plate was slightly centrifuged and incubated at 50°C for 50 minutes and at 85°C for 5 minutes using a thermal cycler to synthesize cDNA.

Next, cDNA prepared from one cell was subjected to PCR using primers for cloning. One µl of cDNA was dispensed into each of four 0.2-ml PCR tubes (#1 to #4) for each sample, and 1 µl of 10 × PCR buffer II (Mg⁺), 1 µl of 2.5 mM dNTP mix, 5.9 µl of dH₂O, 0.1 µl of LA-Taq polymerase (TaKaRa LA-Taq (R) Hot Start version; from Takara Bio Inc.), 0.5 µl of a forward primer (10 µM), and 0.5 µl of a reverse primer (10 µM) were added to each tube, which was then subjected to PCR reaction using a thermal cycler (Gene Amp^{R} PCR System 9700; from Applied Biosystems). Tubes #1, #2, #3 and #4 were used for the PCR reaction of the β-actin gene, the human IgG heavy chain region gene, the human IgG light chain κ region gene and the human IgG light chain λ region gene, respectively. The PCR reaction conditions for each tube were as follows:
Tube #1: 94°C for 5 minutes, (94°C for 15 seconds, 68°C for 2 minutes) × 55 cycles, 72°C for 5 minutes;
Tube #2 and tube #3: 94°C for 5 minutes, (94°C for 15 seconds, 68°C for 1 minutes) × 55 cycles, 72°C for 5 minutes; and
Tube #4: 94°C for 5 minutes, (94°C for 15 seconds, 60°C for 30 seconds) × 40 cycles, 72°C for 5 minutes.

After PCR reaction, the resultant reaction solution was subjected to electrophoresis using 1.5% agarose gel, and a desired band was identified with ethidium bromide staining. The size of bands of PCR products amplified with primer sets for human IgG region gene region amplification was identified, and 2 samples of DNA for each primer set were extracted and purified from gel. Specifically, gels of amplification band portions for the PCR reaction tubes #2, #3 and #4 were cut out using a scalpel and transferred to 1.5-ml sample tubes, and the cut-out gels were weighed. Converting at the rate of 1 µl to 1 mg, a 3-fold volume of Buffer Q × 1 and 17.2 µl of QIAEX II Suspension were added to each cut-out gel. This was mixed thoroughly with a vortex, placed on a heat block set at 50°C in advance and subjected to vortex mixing every 2 minutes, resulting in treatment for 10 minutes in total. Centrifugation was then carried out at 10,000 × g and room temperature for one minute and the supernatant was removed. Again, 500 µl of Buffer Q × 1 was added to the precipitate, which was then mixed using a vortex and centrifuged at 10,000 × g and room temperature for one minute. The supernatant was removed, and 500 µl of Buffer PE to which ethanol was added in advance was added to the precipitate, which was then mixed using a vortex and centrifuged at 10,000 × g and room temperature for one minute, followed by removing the supernatant. Again, 10,000 × g was applied to the precipitate, which was then mixed using a vortex and centrifuged at 10, 000 × g and room temperature for one minute, followed by removing the supernatant. Each sample tube was placed while opening the lid thereof in a clean bench for 15 minutes to dry the precipitate. Buffer PE (Min Elute^{™} Reaction Cleanup Kit ; from QIAGEN) (20 µl) was added to the precipitate, which was then mixed using a vortex and placed at room temperature for 5 minutes. This was centrifuged at 10,000 × g and room temperature for one minute, the supernatant was recovered into another 1.5-ml sample tube, and 20 µl of Buffer PE was again added to the precipitate. This was mixed using a vortex, placed at room temperature for 5 minutes, centrifuged at 10,000 × g and room temperature for one minute, and added to the supernatant previously recovered.

To a total 40 µl of the recovered solution was added 300 µl of Buffer ERC (MinElute^{™} Reaction Cleanup Kit; from QIAGEN), which was then mixed using a vortex. A total amount of the mixed solution after being identified to have a yellow color was applied into MinElute column (MinElute^{™} Reaction Cleanup Kit; from QIAGEN) set in a 2-ml collection tube (MinElute^{™} Reaction Cleanup Kit; from QIAGEN) and centrifuged at 10, 000 × g and room temperature for one minute. After discarding the effluent, the column was again returned to the collection tube, and 750 µl of Buffer PE to which ethanol was added in advance was added thereto, which was centrifuged at 10,000 × g and room temperature for one minute. After discarding the effluent, the column was again returned to the collection tube, and centrifugation was carried out at room temperature and 22,000 × g for one minute. Droplets attached to the brim of the column were removed using a micropipette, and the column was set in a new 1.5-ml sample tube. Thereto was added 10 µl of Buffer EB (MinElute^{™} Reaction Cleanup Kit; from QIAGEN), which was then placed at room temperature for one minute and then centrifuged at 10, 000 × g and room temperature for one minute, followed by recovering a purified DNA fragment.

The PCR fragment was inserted into pCR4-TOPO-TA Plasmid vector to produce a plasmid DNA. Salt Solution (TOPO TA Cloning^{R} Kit for Sequencing; from Invitrogen) of 1 µl of the DNA fragment and 1 µl of TOPO^{R} Vector (TOPO TA Cloning^{R} Kit for Sequencing; from Invitrogen) were mixed on ice. The mixture was reacted at room temperature for 5 minutes, again returned onto ice, and used for transformation. The plasmid DNA was introduced into DH5α competent cells (Competent high DH5α; from Toyobo Co., Ltd.). The plasmid DNA was added 2 µl by 2 µl to 20 µl of thawed DH5α competent cells, which was gently mixed using an end of a tip. The mixture was placed on ice for 30 minutes and then treated at 42°C for 30 seconds using a heat block. This was again placed on ice for 2 minutes for cooling, to which 250 µl of SOC medium was then added, followed by shaking culture at 37°C for one hour. While the shaking culture was carried out, 50 µl each of 0.1 M IPTG (isopropyl β-D-1-thiogalactopyranoside; from Sigma) and 0.1 M X-Gal (5-bromo-4-chloro-3-indolyl β-D-galactopyranoside; from Sigma) were coated on 2 × YT solid medium containing 50 µg/ml of kanamycin. The cultured sample (100 µl) was seeded on the prepared solid medium and cultured 37°C overnight. After identifying the appearance of colonies on the solid medium, this was allowed to stand at 4°C for 5 hours. White colonies were marked, poked with an end of a tip, and placed and slightly rinsed in a 96-well plate containing 50 µl of sterilized water. This was treated at 95°C for 5 minutes using a thermal cycler and then slightly centrifuged, and 2 µl thereof was placed in a fresh plate well. Subsequently, 1 µl of 10 × PCR buffer II (Mg⁺), 0.8 µl of 2.5 mM dNTP Mix, 6.11 µl of dH₂O, 0.05 µl of LA-Taq polymerase (TaKaRa LA-Taq (R) Hot Start version; from Takara Bio Inc.), 0.02 µl of 100 µM M13 forward primer, and 0.02 µl of 100 µM M13 reverse primer were added thereto, and the plate was slightly centrifuged. After thermal denaturation at 94°C for one minute using a thermal cycler, Gene Amp^{(R)} PCR System 9700, PCR reaction was performed under reaction conditions in which the cycle of reaction at 94°C for 10 seconds, 50°C for 10 seconds, and 68°C for 2 minutes was repeated 35 times. PCR products (tube #2: 1.6 Kbp, tube #3: 0.9 Kbp, and tube #4: 0.9 Kbp) in PCR reaction solutions were identified by electrophoresis using 1.5% agarose gel.

Colonies in which the insertion of the PCR-amplified fragment into the vector was identified were selected and cultured with shaking at 37°C overnight in a 2 × YT liquid medium containing 3.5 ml of 50 µg/ml kanamycin. The cultured sample (1.8 ml) was placed in a 2-ml sample tube and centrifuged at 1,000 × g for 10 minutes. The supernatant was discarded, and 250 µl of Buffer A1 (NucleoSpin^{(R)} Multi-8 Plasmid; from Macherey-Nagel) was added to the precipitate, which was then mixed using a vortex. Subsequently, 250 µl of Buffer A2 was added thereto, which was then mixed by inversion and allowed to stand at room temperature for 5 minutes to lyse the cells. Buffer A3 (350 µl) was added thereto and mixed by inversion, which was then centrifuged at 4°C and 14, 000 × g for 10 minutes. The supernatant was transferred to NucleoSipn^{(R)} Plasmid Binding Strips set in NucleoVac vacuum manifold. The solution was passed through the silica membrane by suction at 400 mbar for one minute to produce DNA binding. The silica membrane was washed by adding 600 ml of Buffer AW and passing the solution therethrough by suction at 400 mbar for one minute and then adding 900 ml of Buffer A4 and passing the solution therethrough by suction at 400 mbar for one minute. The silica membrane was again washed by adding 900 ml of Buffer A4 and passing the solution therethrough by suction at 400 mbar for one minute. The silica membrane was dried by suction at 600 mbar for 15 minutes. The plasmid DNA was recovered by subjecting the NucleoVac vacuum manifold to replacement with NucleoSipn^{R} MN Tube Strips for recovery, adding 120 µl of Buffer AE to the membrane, allowing the mixture to stand for one minute, and suctioning it at 400 mbar for one minute. To 3 µl of the recovered plasmid DNA solution were added 1 µl of 10 × H Buffer, 5 µl of dH₂O, and 1 µl of EcoRI (from Toyobo Co., Ltd.), which was then treated at 37°C for one hour. DNA digestion fragments (tube #2: 1.4 Kbp, tube #3: 0.7 Kbp, and tube #4: 0.7 Kbp) in the enzymatic reaction solution were identified by electrophoresis using 1.5% agarose gel; absorbance for each plasmid sample was measured to calculate the DNA concentration; and 100 µg/µl of a plasmid DNA diluted solution was prepared.

The clone obtained was sequenced by the cycle sequencing method. The DNA diluted solution prepared in Example 11 was added in amounts of 6 µl/well to 3 wells for tube #2 (#2-1, #2-2 and #2-3), 2 wells each for tubes #3 and #4 (#3-1 and #3-2 and #4-1 and #4-2) in a 96-well plate placed on ice. Subsequently, 3 µl of 5 × Sequencing Buffer (BigDye^{R} Terminator v3.1 Cycle Sequencing Kit; from Applied Biosystem), 2 µl of BigDye^{R} Terminator Pre mix (BigDye^{R} Terminator v3.1 Cycle Sequencing Kit; from Applied Biosystem), 8 µl of dH₂O, and 1 µl of 3.2 µM primer were added to each well, and slightly centrifuged.

The primers used for the samples are M13 reverse primer for sample #2-1, M13 forward primer for sample #2-2, HuIGCH-seq001 for sample #2-3, M13 reverse primer for sample #3-1, M13 forward primer for sample #3-2, M13 reverse primer for sample #4-1, and M13 forward primer for sample #4-2. After thermal denaturation at 94°C for one minute using a thermal cycler, Gene Amp^{(R)} PCR System 9700, PCR reaction was performed under reaction conditions in which the cycle of reaction at 94°C for 10 seconds, 50°C for 5 seconds, and 68°C for 4 minutes was repeated 25 times. By the time the reaction was completed, Sephadex G-50 (Sephadex^{™} G-50 Superfine; from GE Healthcare) and 300 µl of sterilized water were added to wells of MultiScreen^{™} HV-plate (MultiScreen^{R} HV-plate; from Millipore) and allowed to stand at room temperature for 2 hours. After sufficient hydration, centrifugation was carried out at room temperature and 1,100 × g for 5 minutes, and the effluent was discarded. The MultiScreen^{™} HV-plate was subjected to replacement with a fresh 96-well plate (ASSAY PLATE 96 well round bottom; from Iwaki); a total amount of the reaction solution was applied to wells; and centrifugation was carried out at room temperature and 1,100 × g for 5 minutes to recover the sample.

A total amount of the purified sample was transferred to a 96-well plate for sequencing (MicroAmp^{R} Optical 96-well Reaction Plate; from Applied Biosystem); in addition, 17.2 µl of sterilized water was added to the preceding well and a total amount thereof was added to the same sample while washing the well therewith. Using x3130/Genetic Analyzer (from Applied Biosystem), sequences of 6 to 8 clones were read for each sample, and the resultant sequences were subjected to multiple alignment analysis. For a base different between clones, the base which more clones have was regarded as correct sequence to determine the base sequence for each sample.

As a result of the above cloning, as shown in Table 1, 12 clones of IgG antibody genes were successfully isolated from 5 cases (MEL-008, MEL-014, MEL-016, MEL-018Pre, and MEL-018Post) of melanoma patient-derived EBV-B cell lines (at a single-cell level). For five of these clones, the expression of single-chain recombinant antibodies has been identified and the antibodies are now at a stage of purification.

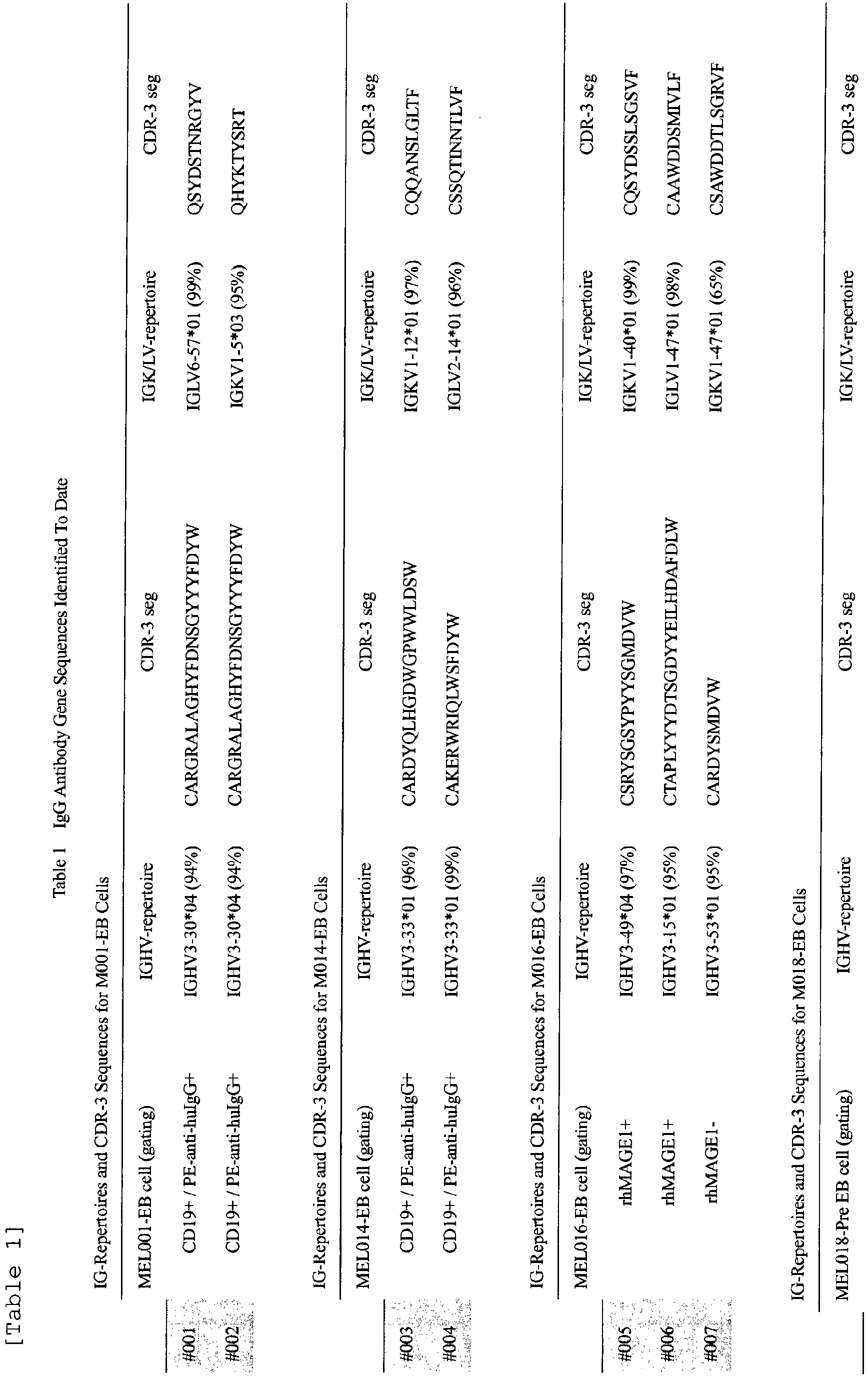

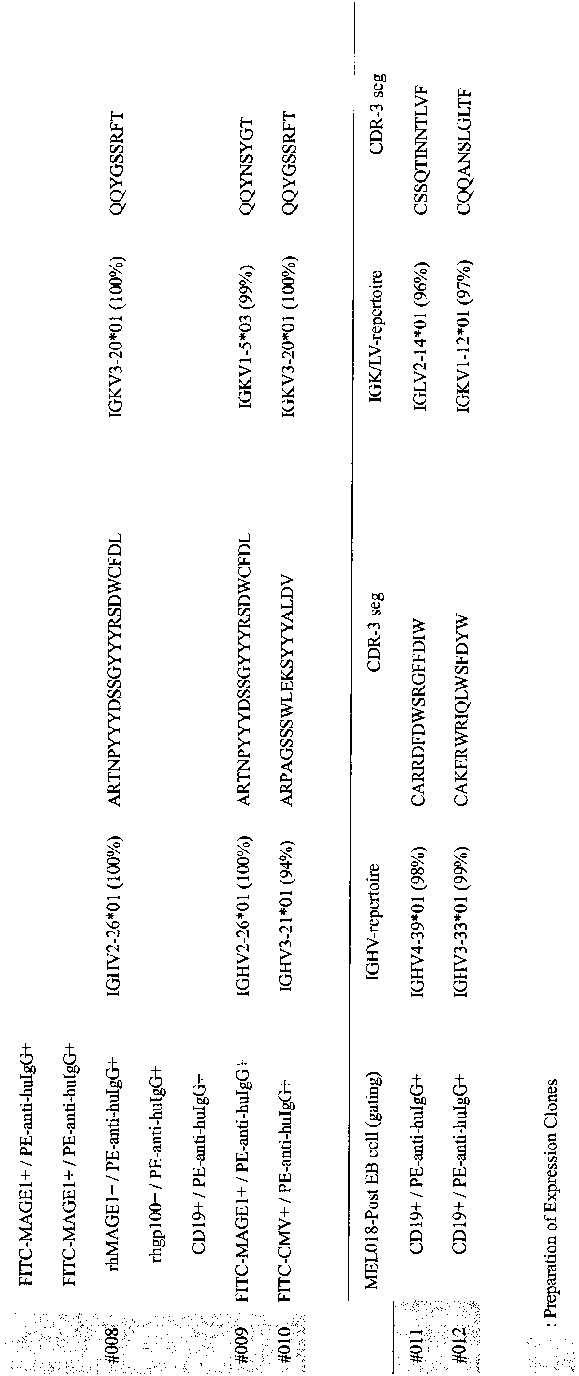

### Example 6

### [Expression/Purification and Functional Analysis of Single-Chain Recombinant Antibody scFv Protein]

Expression of MEL-018scFv: Of the IgG genes cloned in Example 5, the gene sequence for #008 (MEL018Pre) was used to incorporate the genes VH and VL of the variable regions of the IgH chain and the IgL chain into a plasmid for single-chain antibody gene expression (p0Z1, a pUC119-derived self-made vector) to express MEL-018 scFv (single chain recombinant antibody) in Escherichia coli (Figure 4). FLAG tag and His tag were linked to the C-terminus of the antibody gene; flag and His tag were used for the detection of protein and purification, respectively. A method for culturing Escherichia coli will be specifically described below. Pre-culture was performed at 37°C and 250 rpm overnight in a 2 × YT medium (ampicillin), and main culture was carried out at 37°C and 250 rpm for 4 hours in a 2 × YT medium (ampicillin). Thereafter, IPTG (1 mM) was added for expression induction, and culture was further performed for 20 hours. After centrifuging the cultured bacterium (4°C, 8,000 rpm, 10 minutes) for the harvest thereof, cells were disrupted (by treatment with 10 ml of BugBuster protein extraction reagent and 1 µl of Benzonase nuclease (both from Novagen) at 25°C for 30 minutes), and an insoluble fraction was removed by centrifugation (4°C, 15,000 rpm, 30 minutes) to collect the supernatant (E. coli soluble fraction).

Purification of MEL-018 scFv Antibody: Using His tag as a marker, Metal chelate affinity purification employing a Ni Sepharose column was carried out. Then, two-step purification was performed by anion exchange chromatography (HiTrap QFF column). In addition, for the purpose of evaluating the specificity of the finally purified MEL-018 scFv, western blotting was performed using the GST-labeled recombinant MAGE1 protein (543 aa, 59.74 kDa) as an antigen. A purified primary antibody (MEL-018 scFv) was diluted by 1/1,000 and reacted for 2 hours, and then a secondary antibody (anti-FLAG M2 monoclonal antibody) was diluted by 1/2,000 and reacted for 2 hours. A mouse anti-human MAGE1 monoclonal antibody (from Abnova) was used as a control antibody. ECLplus reagent (from GE Healthcare) was reacted for 10 minutes to detect a signal.

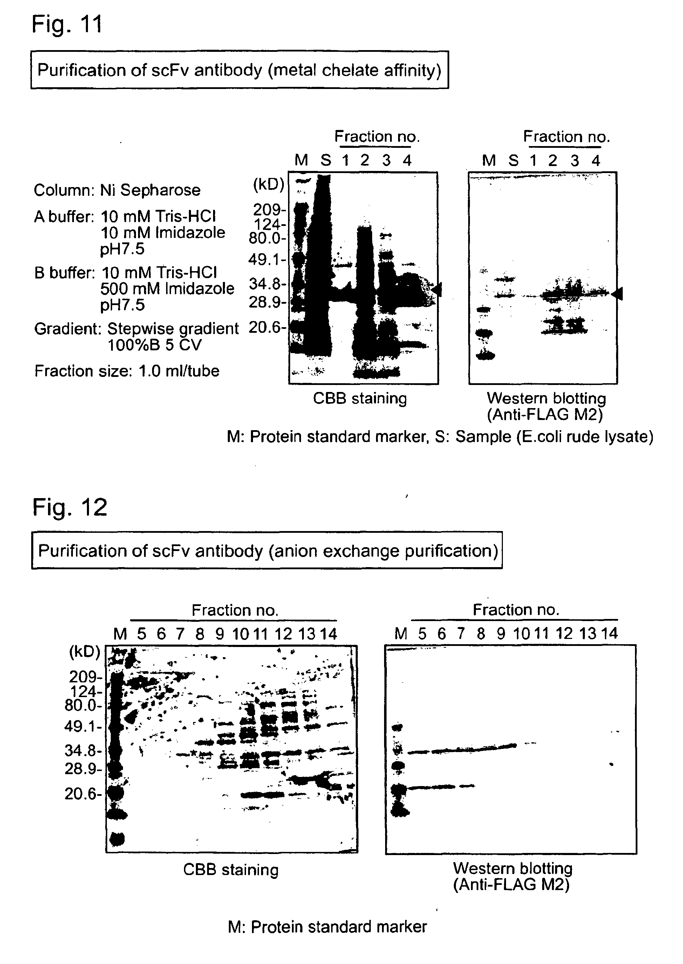

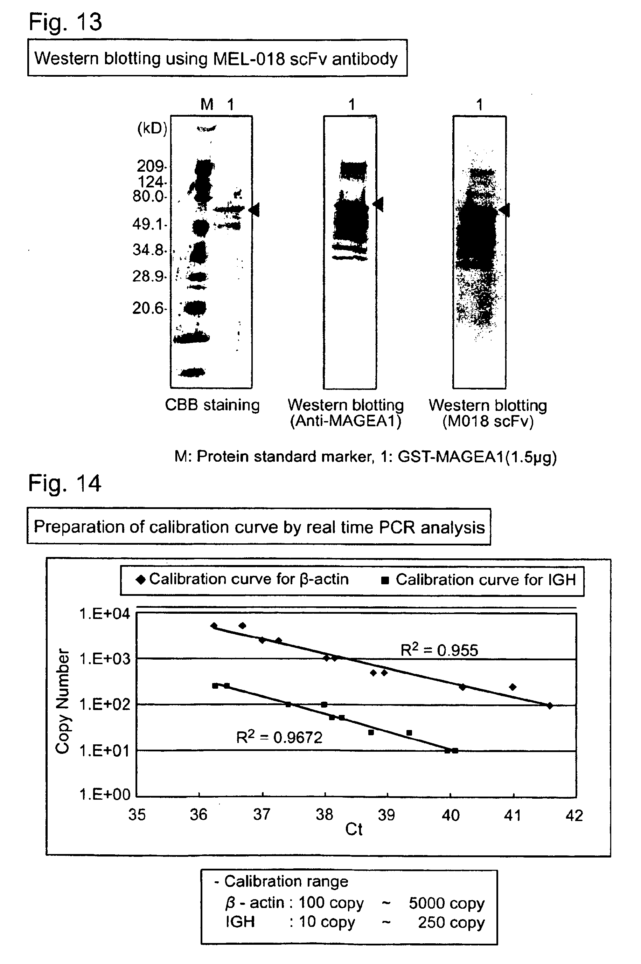

The results of the above experiment are shown in Figure 11. The results of metal chelate affinity purification of a soluble fraction containing MEL-018scFv antibody are shown. The recovery of an antibody protein having a size of 30 kd in elution Fr. 2 to 4 was identified. As shown in Figure 12, as a result of performing anion exchange chromatography as a second step, the scFv antibody was recovered in elution Fr. 6 to 9. In addition, as shown in Figure 13, as a result of western blotting, it could be identified that the scFv antibody like the mouse antibody specifically recognized the recombinant MAGE1 protein (a band around 60 Kd).

### Example 7

### [Quantitation of IgG Antibody Gene of EBV-B Cells at Single-Cell Level]

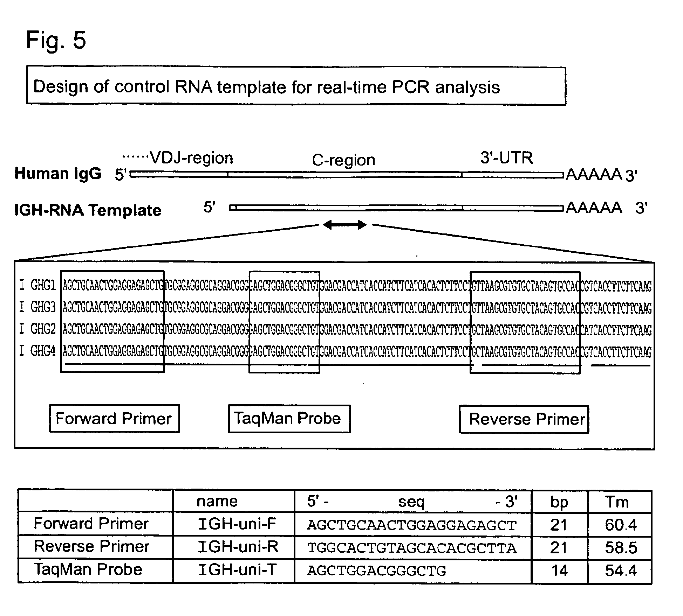

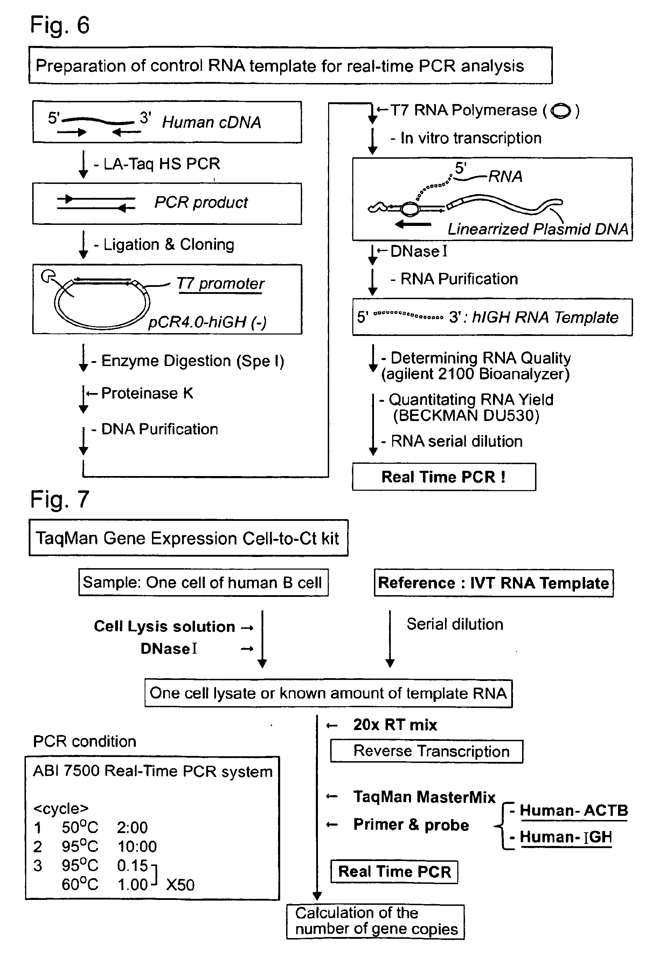

The IgG antibody gene of immortalized B cells was quantified at a single-cell level by the real-time PCR method. Probe (TaqMan) primers targeting a conserved region of the Fc segment of the IgG antibody were designed, and the mRNA of a partial sequence of the Fc segment for preparing a calibration curve was synthesized in vitro (Figures 5 to 7). Normal B cells separated with CD19 microbeads in the same melanoma case and an immortalized B cell line were subjected to quantitative real-time PCR, and the number of β-actin and IgG genes per cell was measured and compared.

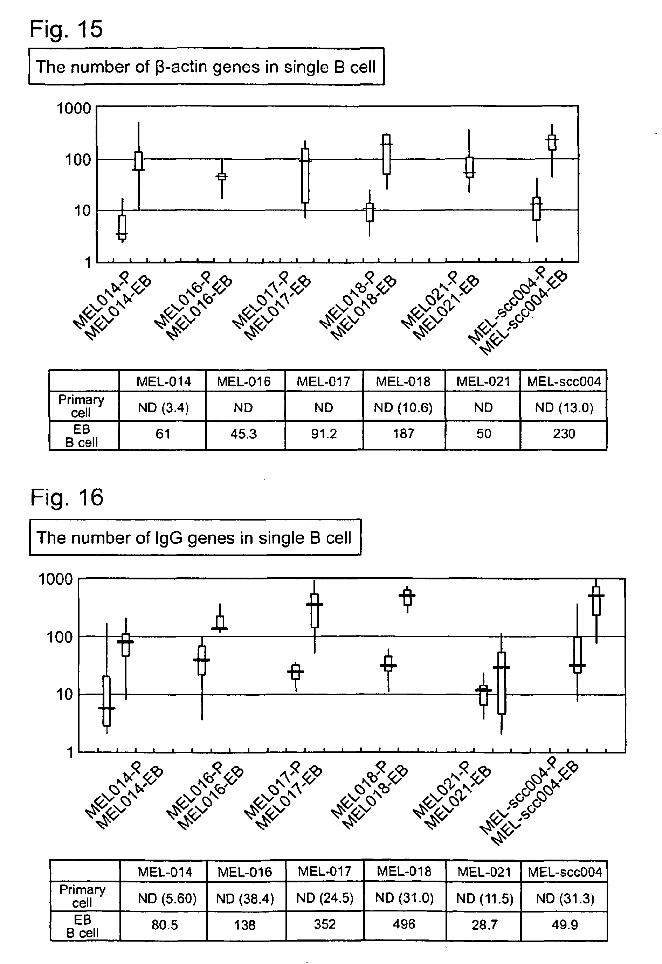

The results of the above quantitation using the real-time PCR method are shown in Figures 14 to 16. The in vitro synthesized β-actin and human IgG mRNAs were serially diluted, and PCR amplifications were performed to prepare calibration curves used for quantitation of the number of their copies (Figure 14). From these calibration curves, the quantifiable number of copies per cell in the real-time PCR system was found to be 100 to 5,000 for β-actin and 10 to 250 for human IgG. As a result of performing a real-time PCR analysis using 6 EB-B cell lines derived from melanoma patients, the number of β-actin gene copies per cell was 110.7 on average in immortalized B cells; although this gene was not amplified in normal B cells, the number of such copies was speculated to be around 10 when converted from amplification data from 10 cells (Figure 15). On the other hand, the number of IgG antibody gene copies of EB-B cell was 265.7 copy/per cell on average, whereas the amplification was not detected in normal B cells. Thus, similarly when converted from data from 10 cells, the number of such copies was speculated to be 23.7/cell on average in normal B cells. From the above results, it was identified that the number of IgG antibody gene copies in EB-B cells was amplified 10 times or more that in normal B cells (Figure 16).

### Example 8

### [Antibody Gene Analysis at Single-Cell Level Using Non-Immortalized B Cells]

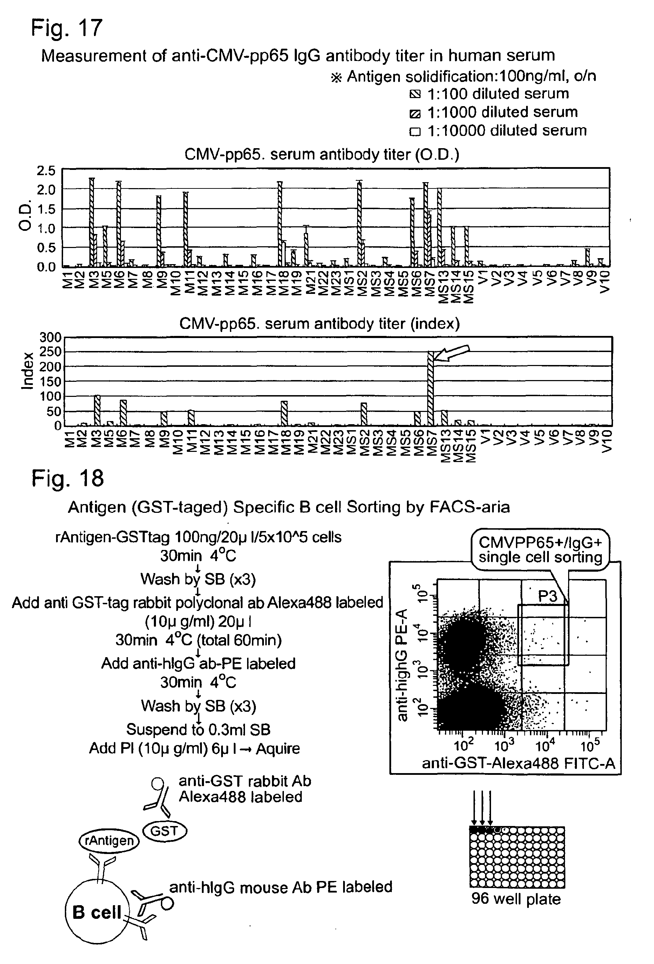

Sera were collected from cancer patients and healthy subjects, and the anti-CMVpp65 antigen-specific IgG antibody titer in each serum was measured. The results are shown in Figure 17. The MEL-SCC007-derived B cells found to have the highest antibody titer were used for subsequent identification of an antibody gene.

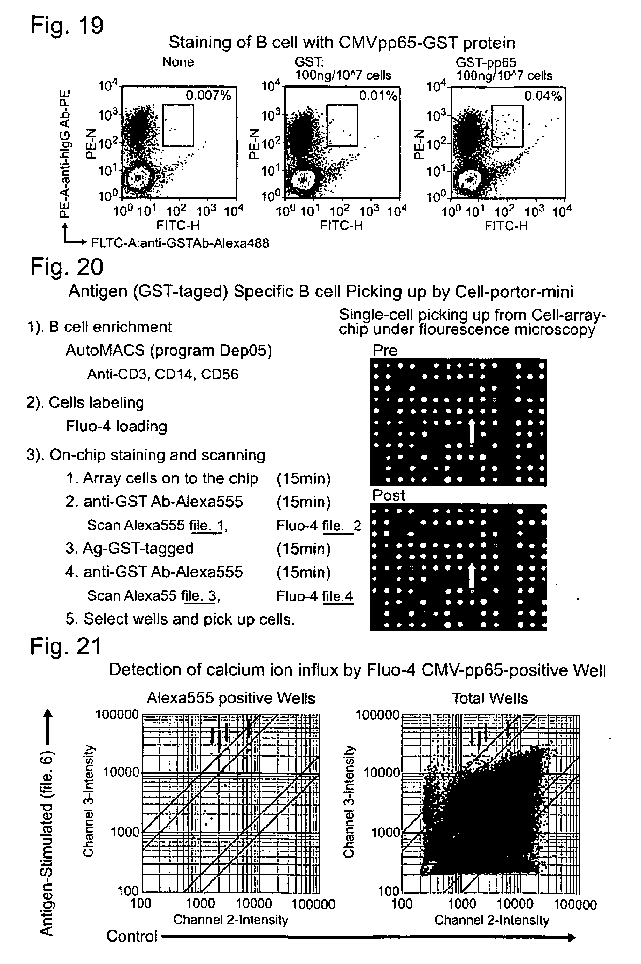

Subsequently, B cells were stained with GST-labeled CMVpp65 antigen protein, Alexa488-labeled anti-GST antibody, and a PE-labeled anti-human IgG antibody (Figure 18). Specifically, the recovered B cells were first washed three times with a phosphate-buffered saline containing 0.5 ml of 2% calf serum and 0.1% sodium azide (FCS-PBS) (200 to 400 × g, 4 minutes, 4°C), and 5 × 10⁵ cells were dispensed into 1. 5-ml tubes and adjusted to a volume of 20 µl with FCS-PBS. To each dispensed cells were added 20 µl of GST-labeled CMVpp65 antigen protein adjusted to 100 ng/20 µl, which was then reacted at 4°C for 30 minutes under shielding the light. The resultant cells were washed three times with 0.5 ml of FCS-PBS (400 × g, 2 minutes, 4°C), and 20 µl of 10 µg/ml Alexa488-labeled rabbit anti-GST polyclonal antibody (from Invitrogen) was added thereto, which was then reacted 4°C for 30 minutes under shielding the light. Five µl each of PE-labeled anti-human immunoglobulin antibody (from BD) and APC-labeled anti-human CD19 antibody were added thereto, which was then reacted 4°C for 30 minutes under shielding the light. The resultant cells were washed three times with 0.5 ml of FCS-PBS (400 × g, 2 minutes, 4°C), floated in 0.5 ml of FCS-PBS, and transferred to a 5-ml tube for the incorporation of a flow cytometer. The tube was stored on ice under shielding the light before cell incorporation. 10 µg/ml of propidium iodide (10 µl) was added in order to distinguish dead cells, if necessary. Using FACS-aria, cells stained with all of an APC-labeled antibody, an Alexa488-labeled antibody, and a PE-labeled antibody were dispensed into wells of a 96-well plate on one cell by one cell basis. B cells having been stained with both of CMV antigen/IgG antibody in this manner account for 0.04% of the total CD19-positive B cells and were subjected to single cell sorting using FACS-aria. The results are shown in Figure 19. In the case of staining with 100 ng/10⁷ cells of CMVpp65 antigen protein, the percentage of CMVpp65⁺/IgG⁺ cells was 0.04% (0.01% for only GST protein). The number of the cells captured on one cell by one cell basis for use in RT-PCR was 57 in total.

### Example 9

### [Capture and Stain Identification of B Cells Using Cell Microarray]

As in Example 8, B cells from cases in which the serum antibody titer to CMVpp65 was increased were used to perform the stain identification and capture of CMVpp65 antigen-positive B cells with cell microarray (Figure 20). B cells were sorted by negative selection using a combination of anti-CD3, CD14 and CD56 antibodies (AutoMACS; from Miltenyi). The B cells were stained by reaction with 0.5 to 1 µM Fluo-4 reagent at 37°C for 40 minutes. The B cells stained with Fluo-4 were added on a microchip, allowed to stand for 15 minutes, and adapted to be placed in wells on one cell by one cell basis. To perform background staining, Alexa555-labeled rabbit anti-GST polyclonal antibody was added thereto, which was then reacted at 4°C for 15 minutes. Image data for Alexa555 and Fluo-4 were obtained using a highly sensitive scanner (SC@ Scanner, from SC World Inc.). GST-labeled CMVpp65 antigen protein was added thereto, which was then reacted at 4°C for 15 minutes; and Alexa555-labeled rabbit anti-GST polyclonal antibody was again added thereto, which was then reacted at 4°C for 15 minutes. Here, Image data for Alexa555 and Fluo-4 were obtained. The resultant image data were analyzed using a software dedicated to analysis (TIC-Chip Analysis; from SC World Inc.) to identify B cells stained with both CMVpp65 antigen (Alexa555) and Fluo-4. An automatic single-cell capture device (Cell Porter mini; Sugino Machine) was used to separate the B cells into PCR tubes on one cell by one cell basis. The typical results are shown in Figure 21. Among 24 × 10⁴ CD19⁺ B lymphocytes on the cell chip, 20 CMVpp65 antigen (Alexa555)⁺/Fluo-4⁺ cells were identified and included 5 cells whose intracellular calcium was increased by the addition of the antigen. The number of the cells captured on one cell by one cell basis for use in RT-PCR was 67 in total.

### Example 10

### [Single Cell RT-PCR Cloning]

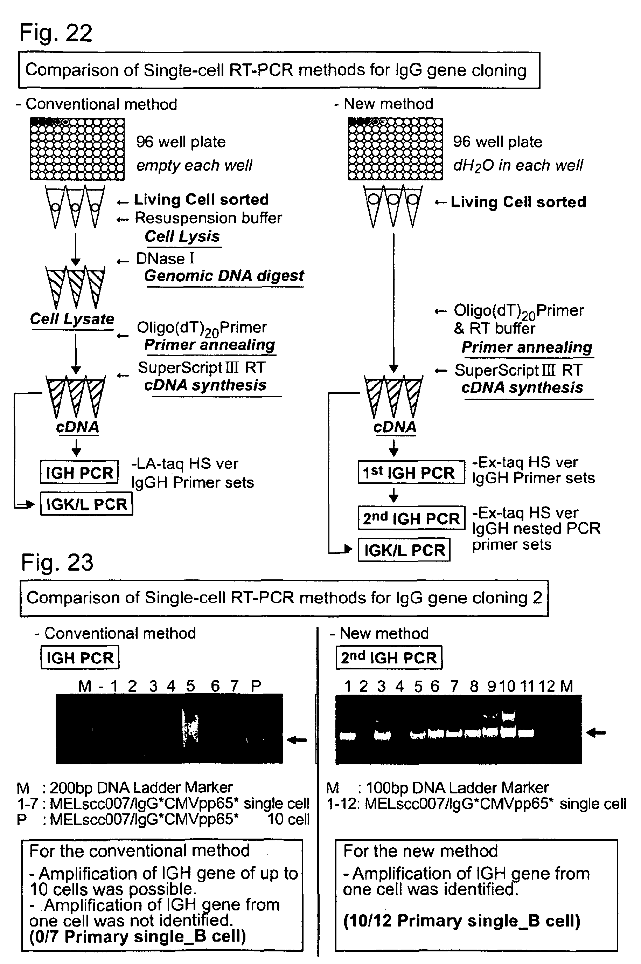

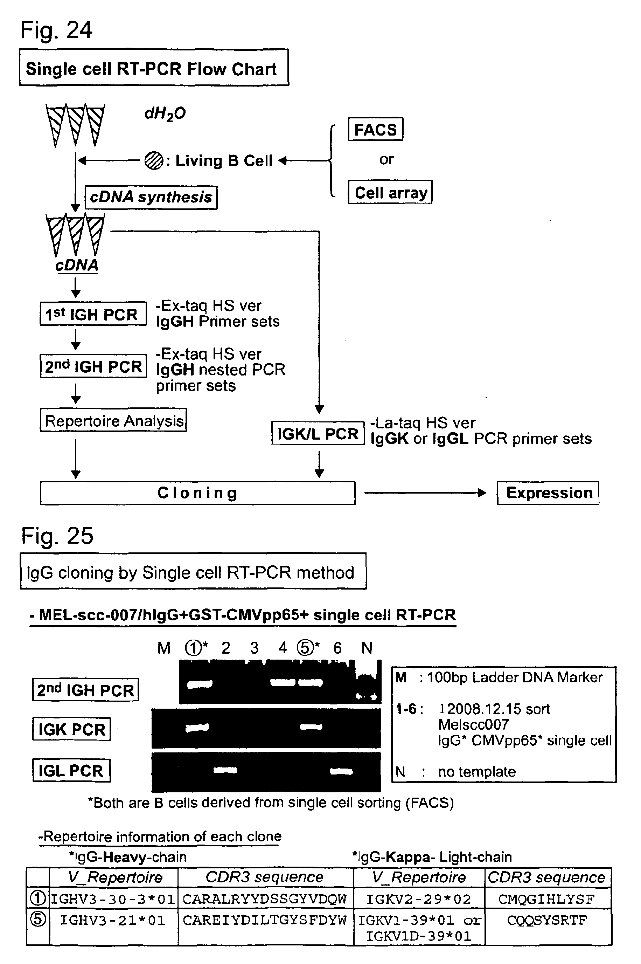

Single cell RT-PCR cloning was then carried out (Figures 22 and 25). Here, single cell RT-PCR cloning was performed in which the following 4 points were improved compared to that in the above Example 5 (Figure 22):
1. Sterilized water (5 µl) containing yeast transfer RNA as a carrier was added to each well of a 96-well plate or each 0.2-ml tube for PCR and thereby adapted to receive the separated live cells;
2. DNase treatment and cell-lysing treatment were omitted, considering that the cell-lysing treatment was unnecessary because cell rupture by osmotic pressure and content release occurred the instant cells contacted the sterilized water.
3. A solution (6 µl) of the synthesized cDNA was used to perform IGH-PCR. In the method of Example 5, the cDNA solution could be introduced into the PCR reaction only under conditions of 1 µl/30 µl of the total PCR reaction solution; thus, this system (6 µl/12 µl of the total PCR reaction solution) could be expected to have higher detection sensitivity than that of the previous one.
4. Nested PCR method was adopted in order to improve specificity and amplification efficiency. Both 1^{st}-PCR and 2^{nd} PCR used Ex-taq HotStart ver; in 1^{st}-PCR, primers corresponding to each repertoire used the same ones as those in Example 5.

Reverse transcription reaction directly targeting a single cell was carried out as follows. Sterilized water (5 µl) containing yeast transfer RNA (from Ambion) as a carrier and RNase OUT™ (from Invitrogen) were placed in a 96-well plate (MicroAmp^{R} Optical 96-Well Reaction Plate; from Applied Biosystem) or 0.2-ml PCR tubes, and the sorted cells were separated thereinto on one cell by one cell basis using FACS or Cell porter mini. The 96-well plate or 0.2-ml tubes receiving the cells were slightly centrifuged and placed on ice, and 10 × PCR buffer II (0.80 µl), 25 mM MgCl₂ (0.48 µl), 0.1 M DTT (0.40 µl), 40 U/µl RNase OUT™ (0.16 µl), 50 mM Oligo (dT)₂₀ (from Invitrogen) (0.16 µl), 10 mM dNTPmix (0. 16 µl), and dH₂O (0.84 µl) were added to each well, which was then treated ashing thermal cycler at 70°C for 90 seconds using a thermal cycler (Gene Amp^{R} PCR System 9700; from Applied Biosystems). After thermal treatment, the 96-well plate or 0.2-ml PCR tubes were quickly transferred onto ice and allowed to stand for 2 minutes. The 96-well plate or 0.2-ml PCR tubes were slightly centrifuged and then again transferred onto ice, and RNase OUT^{™} (40 U/µl), 0.05 µl of Super Script^{™} III RT (200 U/µl; from Invitrogen), 0.40 µl and 1.35 µl of dH₂O were added to each well, which was then mixed by pipetting. The 96-well plate was slightly centrifuged and then treated using thermal cycler at 50°C for 50 minutes and 70°C for 10 minutes to synthesize DNA. In addition, 10 × PCR buffer II (0.20 µl), MgCl₂ (0. 12 µl), RNase H (0.30 µl; from Invitrogen), and dH₂O (1.38 µl) were added thereto, which was then thoroughly mixed and treated at 37°C for 15 minutes and 70°C for 10 minutes to prepare 12 µl of a cDNA solution.

### Example 11

### [1^{st} PCR of Human Antibody Heavy Chain Region Gene (IGH)]

Using each of the synthesized cDNA solutions as a template, 1^{st} PCR of a human antibody heavy chain region gene (IGH) was carried out as follows. Specifically, 0.2-ml tubes for PCR were provided and placed on ice, and 6 µl of the cDNA solution prepared in Example 9 was placed in each 0.2-ml tube for PCR. Subsequently, 10 × Ex Taq buffer II (2.0 µl), 2.5 mM dNTP Mix (2.0 µl), dH₂O (13.8 µl), Ex-Taq Hot Start version (0.2 µl; from Takara Bio Inc.), 10 µM forward primer mix [HuIGHV_1 to 24] (1.0 µl), and 10 µM reverse primer mix [HuIGHC_1 to 2] (1.0 µl) were added thereto, which was then slightly centrifuged. The base sequences of primers specific to the human igG heavy chain genes used here are shown in SEQ ID NO: 1-26, respectively. The 0.2-ml tubes for PCR were set in a thermal cycler (Gene Amp^{R} PCR System 9700), and reaction was performed using the program of (95°C for 15 seconds, 68°C for 1 minute, and 72°C for 2 minutes) × 30 cycles and 72°C for 5 minutes.

### [2^{nd} PCR of Human Antibody Heavy Chain Region Gene (IGH)]

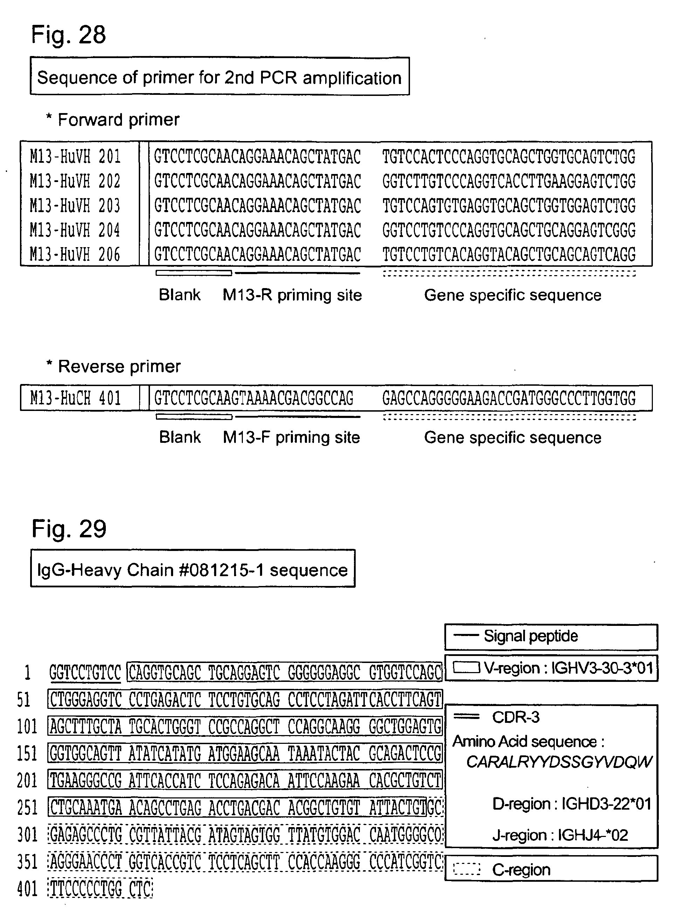

Using each of the PCR products obtained in the above 1^{st} PCR as a template, 2^{nd} PCR was further performed. Specifically, 0.2-ml tubes for PCR were each placed on ice, and 0.5 µl of the 1^{st} PCR reaction product was added to each 0.2-ml tube. Subsequently, 10 × Ex Taq buffer II (2.0 µl), 2.5 mM dNTP Mix (2.0 µl), dH₂O (13.8 µl), Ex-Taq Hot Start version (0.2 µl), 10 µM forward primer mix [M13-HuVH_201 to 206] (1.0 µl), and 10 µM reverse primer [M13-HuCH_401] (1.0 µl) were added thereto, which was then slightly centrifuged. The base sequences of the above primers [M13-HuVH_201 to 206 and M13-HuCH_401] are shown in Figure 28 and SEQ ID NOS: 65 to 70. The 0.2-ml tubes for PCR were set in a thermal cycler, and reaction was performed using the program of (95°C for 15 seconds, 68°C for 1 minute, and 72°C for 1 minute) × 50 cycles and 72°C for 5 minutes.

### [DNA Extraction from PCR Product]

The 2^{nd} PCR reaction solutions thus obtained were each fractionated by electrophoresis to purify a desired PCR fragment. The 2^{nd} PCR reaction solution was subjected to electrophoresis using 1.5% agarose gel to separate bands; after electrophoresis, the gel was stained with ethidium bromide; and after identifying amplification using UV, a gel of an amplified band portion was cut out and transferred to a 1. 5-ml sample tube. For the clone in which the amplification of IGH could be identified, PCR amplifications of the human antibody light chain κ and λ region genes as shown in the following Example 11 were carried out, or a PCR amplified band of a sample for which the amplification of both human antibody heavy chain region gene and human antibody light chain κ or λ region gene was identified was purified. Specifically, the cut-out gel was weighed and, converting at the rate of 1 µl to 1 mg, Buffer QX1 (QIAEXII^{R} Gel Extraction Kit; from QIAGEN) was added in an amount of 3 times that of the gel thereto together with 17.2 µl of QIAEXII Suspension (MinElute^{™} Reaction Cleanup Kit; from QIAGEN), which was then thoroughly mixed using a vortex. The mixture was placed on a heat block set at 50°C in advance and subjected to vortex mixing every 2 minutes, resulting in treatment for 10 minutes in total to completely dissolve the gel. This solution was identified to have a yellow color and then centrifuged at room temperature and 10,000 × g for one minute to remove the supernatant. Again, 500 µl of Buffer QX1 was added to the precipitate, which was then mixed using a vortex and centrifuged at room temperature and 10, 000 × g for one minute. After removing the supernatant, 500 µl of Buffer PE (QIAEXII^{R} Gel Extraction Kit; from QIAGEN) was added thereto, which was then mixed using a vortex. The mixture was centrifuged at room temperature and 10,000 × g for one minute to remove the supernatant, and again, 500 µl of Buffer PE was added to the precipitate, which was then mixed using a vortex and centrifuged at room temperature and 10,000 × g for one minute. After removing the supernatant, the sample tube was placed while opening the lid thereof in a clean bench for 15 minutes to dry the precipitate. Buffer EB (Min Elute^{™} Reaction Cleanup Kit ; from QIAGEN) (20 µl) was added to the precipitate, which was then mixed using a vortex and placed at room temperature for 5 minutes. This was centrifuged at 10,000 × g and room temperature for one minute, the supernatant was recovered into another 1.5-ml sample tube, and 20 µl of Buffer EB was again added to the precipitate. This was mixed using a vortex, placed at room temperature for 5 minutes, centrifuged at 10,000 × g and room temperature for one minute, and the supernatant was recovered in the same tube. To a total 40 µl of the recovered solution was added 300 µl of Buffer ERC (MinElute^{™} Reaction Cleanup Kit; from QIAGEN), which was then mixed using a vortex. A total amount of the mixed solution was applied into MinElute column (MinElute^{™} Reaction Cleanup Kit; from QIAGEN) set in a 2-ml collection tube (MinElute^{™} Reaction Cleanup Kit; from QIAGEN) and centrifuged at 10,000 × g and room temperature for one minute. After discarding the effluent, the column was again returned to the collection tube, and 750 µl of Buffer PE (Min Elute^{™} Reaction Cleanup Kit ; from QIAGEN) to which ethanol was added in advance was added thereto, which was centrifuged at 10,000 × g and room temperature for one minute. After discarding the effluent, the column was again returned to the collection tube, and centrifugation was carried out at room temperature and 22,000 × g for one minute. Droplets attached to the brim of the column were removed using a micropipette, and the column was set in a new 1.5-ml sample tube. Thereto was added 10 µl of Buffer EB (MinElute^{™} Reaction Cleanup Kit; from QIAGEN), which was then placed at room temperature for one minute and then centrifuged at 10, 000 × g and room temperature for one minute, followed by recovering a purified DNA fragment.

### Example 12

### [Experiment of PCR Amplification of Human Antibody Light Chain κ and λ Region Genes]

Two 0.2-ml PCR tubes (#1 and #2) were provided for each sample and placed on ice, and 1 µl of the cDNA solution prepared in Example 10 was placed in each 0.2-ml PCR tube. Subsequently, 10 × PCR buffer II (Mg⁺) (1 µl), 2.5 mM dNTP Mix (1 µl), dH₂O (5.9 µl), LA-Taq Hot Start version (TaKaRa LA-Taq^{R} Hot Start version; from Takara Bio Inc.) (0.1 µl), 10 µM forward primer (0.5 µl), and 10 µM reverse primer (0.5 µl) were added thereto, which was then slightly centrifuged. HuIGKV_1 to 11 mix and HuIGKC_1 (SEQ ID NOS: 27 to 38) were used as primers for light chain κ region amplification in the tube #1 and HuIGLV_1 to 23 mix and HuIGLC_1 to 2 mix (SEQ ID NOS: 39 to 64) were used as primers for light chain λ region amplification in the tube #2. The 0.2-ml PCR tubes were set in a thermal cycler, and reaction was performed using the program of 95°C for 5 minutes, (95°C for 30 seconds, 68°C for 1 minute, and 72°C for 5 minutes) × 55 cycles. After the end of PCR, PCR amplification was identified by the same way as in Example 11, and DNA fragments were purified for samples in which the amplification of IGH and IGK/L derived from the same clone could be identified in a set.

It was demonstrated that the foregoing nested PCR increases the sensitivity of a Single-Cell RT-PCR method. The results of comparing the amplification efficiency of a PCR method of an antibody gene derived from one cell before and after the technological improvement depicted in Figure 22 were shown in Figure 23. The cells are B cells derived from MEL-SCC007 and CMVpp65+/IgG+ cells obtained by single cell sorting with FACSAria. For the method before the improvement, amplification was not identified in any of the 7 cells used, whereas for the new method shown in Example 10, the IGH antibody gene was successfully amplified in 10 of 12 cells and an improvement in efficiency was thereby identified. In addition, for cells in which IGH was successfully amplified, an antibody light chain region gene was also simultaneously amplified from the same cDNA (Figure 25). Same B cells in which the antibody heavy chain region gene (IGH) and the antibody light chain region gene (IGL) were simultaneously amplified were selected and used in the following experiments.

### Example 13

### [Identification of IGH Repertoire]

Subsequently, the base sequences of the human antibody heavy chain gene (IGH) DNA fragments obtained were identified by a PCR-Direct sequence method, and cloning samples were selected. Specifically, 2 µl of the IGH 2^{nd} PCR fragment purified in Example 11 was added to each of two wells (#1 and #2) of a 96-well plate placed on ice. In addition, 5 × Sequencing Buffer (3 µl), BigDye^{R} Terminator Premix (BigDye^{R} Terminator v3.1 Cycle Sequencing Kit; from Applied Biosystem) (2 µl), dH₂O (12 µl), and 3.2 µM of a primer (1 µl) were added thereto, which was then slightly centrifuged. An M13 reverse primer was used for the sample #1 and an M13 forward primer, for the sample #2. Then, 0.2-ml PCR tubes were set in a thermal cycler, and reaction was performed using the program of 95°C for 1 minute, (95°C for 10 seconds, 50°C for 5 seconds, and 68°C for 4 minutes) × 24 cycles. By the time the reaction was completed, Sephadex G-50 (from GE Healthcare) and 300 µl of sterilized water were added to wells of MultiScreen^{™} HV-plate (from Millipore) and the plate was allowed to stand at room temperature for 2 hours. After sufficient hydration, centrifugation was carried out at room temperature and 1,100 × g for 5 minutes, and the effluent was discarded. The MultiScreen^{™} HV-plate was subjected to replacement with a fresh 96-well assay plate (from Iwaki); a total amount of each of the preceding reacted samples was applied to each well; and centrifugation was carried out at room temperature and 1,100 × g for 5 minutes to recover the samples. A total amount of the purified sample was transferred to a 96-well plate for sequencing. In addition, 17.2 µl of sterilized water was added to the preceding well and a total amount thereof was transferred to the 96-well plate to determine the sequence thereof using a DNA sequencer (x3130/Genetic Analyzer; from Applied Biosystem). After sequence determination, alignment analysis with the deposited repertoire was performed using V-QUEST (http://www.imgt.org/IMGT_vquest/share/textes/) to determine the IGH repertoire and CDR-3 sequence of a clone. Based on the information of the IGH repertoire and CDR-3 sequences thus determined, clones having overlapping IGH repertoire and CDR-3 sequences were excluded, and cloning was carried out from the remaining clones by a method as described below.

### [Cloning]

First, a PCR fragment and pCR4.0-TA Plasmid vector were linked using TOPO^{R} TA PCR Cloning Kit for Sequencing (from Invitrogen). Specifically, a 500-µl sample tube was placed on ice, and 4 µl of a DNA fragment obtained in Example 10, 1 µl of Salt Solution, and 1 µl of a plasmid vector were added thereto, which was then mixed by pipetting. The mixture was reacted at room temperature for 5 minutes, again returned onto ice, and used for transformation. DH5α competent cells (from Toyobo Co., Ltd.) were thawed on ice and 20 µl thereof was dispensed into each of other sample tubes. To each of these tubes was added 2 µl of the above plasmid vector reaction solution, which was then gently mixed using an end of a tip. Each tube was placed on ice for 30 minutes, and treated at 42°C for 30 seconds using a heat block. The tube was placed on ice for 2 minutes for cooling, to which 250 µl of SOC medium was then added, followed by shaking culture at 37°C for one hour. The cultured sample (100 µl) was seeded on 2 × YT/Km plate coated with 50 µl each of 0.1 M IPTG (from Sigma) and 0.1 M X-Gal (from Sigma) and cultured at 37°C overnight.

White colonies were selected, inoculated into 3.5 ml of 2 × YT/Km liquid medium, and shaking cultured at 37°C overnight. The culture solution (1.8 ml) after culture was placed in a 2-ml sample tube, which was then centrifuged at 1,000 × g for 10 minutes. The supernatant was discarded, and 250 µl of Buffer A1 (NucleoSpin^{R}Multi-8 Plasmid; from Macherey-Nagel) was added to the precipitate, which was then mixed using a vortex. In addition, 250 µl of Buffer A2 (NucleoSpin^{R}Multi-8 Plasmid; from Macherey-Nagel) was added thereto, which was then mixed by inversion and allowed to stand at room temperature for 5 minutes to lyse the cells. Further, 350 µl of Buffer A3 (NucleoSpin^{R} Multi-8 Plasmid; from Macherey-Nagel) was added thereto, which was then mixed by inversion and centrifuged at 4°C and 14,000 × g for 10 minutes. The supernatant was recovered and transferred to NucleoSipn^{R} Plasmid Binding Strips (NucleoSpin^{R} Multi-8 Plasmid; from Macherey-Nagel) set in NucleoVac vacuum manifold. This was suctioned at 400 mbar for one minute to cause DNA to bind to a silica membrane. The silica membrane was washed by adding 600 ml of Buffer AW (NucleoSpin^{R}Multi-8 Plasmid; from Macherey-Nagel) and passing the solution therethrough by suction at 400 mbar for one minute and then adding 900 ml of Buffer A4 (NucleoSpin^{R} Multi-8 Plasmid; from Macherey-Nagel) and passing the solution therethrough by suction at 400 mbar for one minute. The membrane was washed again by adding 900 ml of Buffer A4 and passing solution therethrough by suction at 400 mbar. After drying the membrane, the plasmid DNA was recovered by subjecting the NucleoVac vacuum manifold to replacement with NucleoSipn^{R} MN Tube Strips (NucleoSpin^{R} Multi-8 Plasmid; from Macherey-Nagel) for recovery, adding 120 µl of Buffer AE (NucleoSpin^{R} Multi-8 Plasmid; from Macherey-Nagel) to the membrane, allowing the mixture to stand for one minute, and suctioning it at 400 mbar for one minute. To 3 µl of the recovered plasmid DNA solution were added 10 × H Buffer (1 µl), dH₂O (5 µl), and 1 µl of EcoRI (from Toyobo Co., Ltd.), which was then treated at 37°C for one hour. The restriction enzyme reaction solution was fractionated by electrophoresis using 1.5% agarose gel, and clones in which inserts (IGH: 0.5 kbp, IGK/L: 0.7 kbp) were identified were selected as samples for sequence analysis. Absorbance for each plasmid sample was measured to calculate the DNA concentration; and 100 ng/µl of a plasmid DNA solution was prepared.

### [Sequencing of Plasmid DNA]

The base sequence of a plasmid DNA was determined by a cycle sequencing method. Specifically, the prepared plasmid DNA diluted solution was added in amounts of 6 µl/well to 3 wells (#1, #2, and #3) for IGH and 2 wells (#4 and #5) for IGK/L in a 96-well plate placed on ice. Subsequently, 5 × Sequencing Buffer (3 µl), BigDye^{R} Terminator Pre mix (2 µl), dH₂O (8 µl), and 3.2 µM primer (1 µl) were added to each well, and slightly centrifuged. The combination of 3.2 µM primer and sample # is as follows:
#1: T7p primer
#2: HuIGCH-seq001 primer
#3: T3p primer
#4: M13 reverse primer
#5: M13 forward primer

A 96-well plate was set in a thermal cycler, and reaction was performed using the program of 94°C for 1 minute, (94°C for 10 seconds, 50°C for 5 seconds, and 68°C for 4 minutes) × 25 cycles. By the time the reaction was completed, Sephadex G-50 was placed in wells of MultiScreen^{™} HV-plate and 300 µl of sterilized water were added thereto and the plate was allowed to stand at room temperature for 2 hours. After sufficient hydration, centrifugation was carried out at room temperature and 1,100 × g for 5 minutes, and the effluent was discarded. The MultiScreen^{™} HV-plate was subjected to replacement with a fresh 96-well assay plate (from Iwaki) ; a total amount of the PCR reaction solution was applied to the wells; and centrifugation was carried out at room temperature and 1,100 × g for 5 minutes to recover the samples. A total amount of the purified sample was transferred to a 96-well plate for sequencer. In addition, 17.2 µl of sterilized water was added to the preceding well and used for washing and then a total amount thereof was transferred to the 96-well plate. Sequence analysis was carried out using x3130/Genetic Analyzer. Sequences of 6 to 8 clones were read for each sample, and the resultant sequences were subjected to multiple alignment analysis; for a base different between clones, the base which more clones have was regarded as correct to determine the base sequence for each sample.

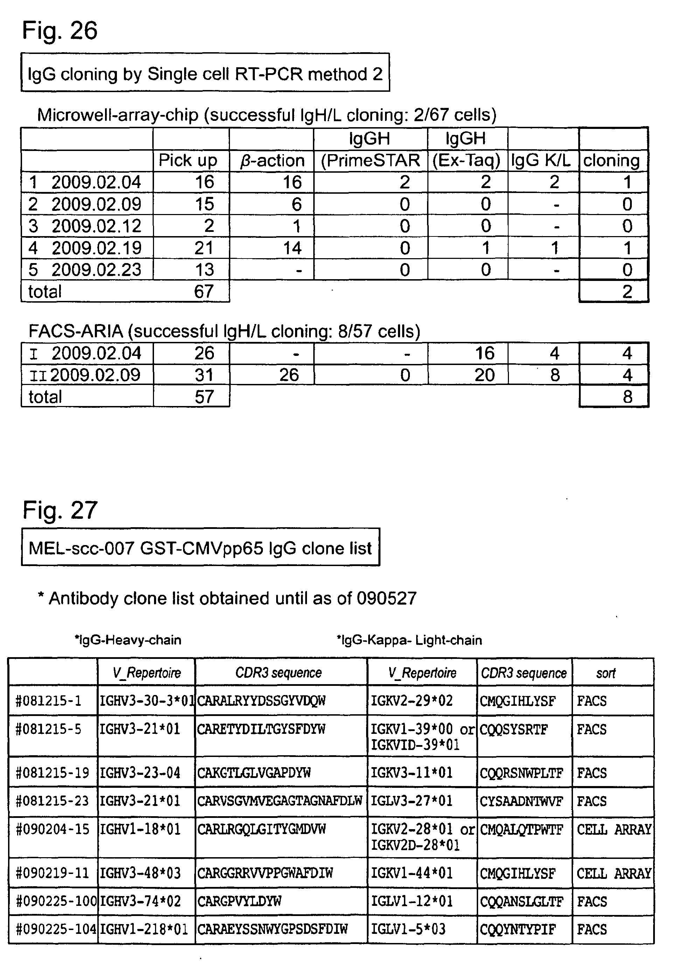

As a result of the above experiment, both antibody genes of IGH/L were successfully cloned in 8 of the 57 cells captured by the single cell sorting method (Figure 26). In the case of using cell microarray, 2 of the 67 cells captured were successful. The reason why more cells were successful for the single cell sorting method was considered to be that IgG-positive cells were sorted in the step of staining B cells.

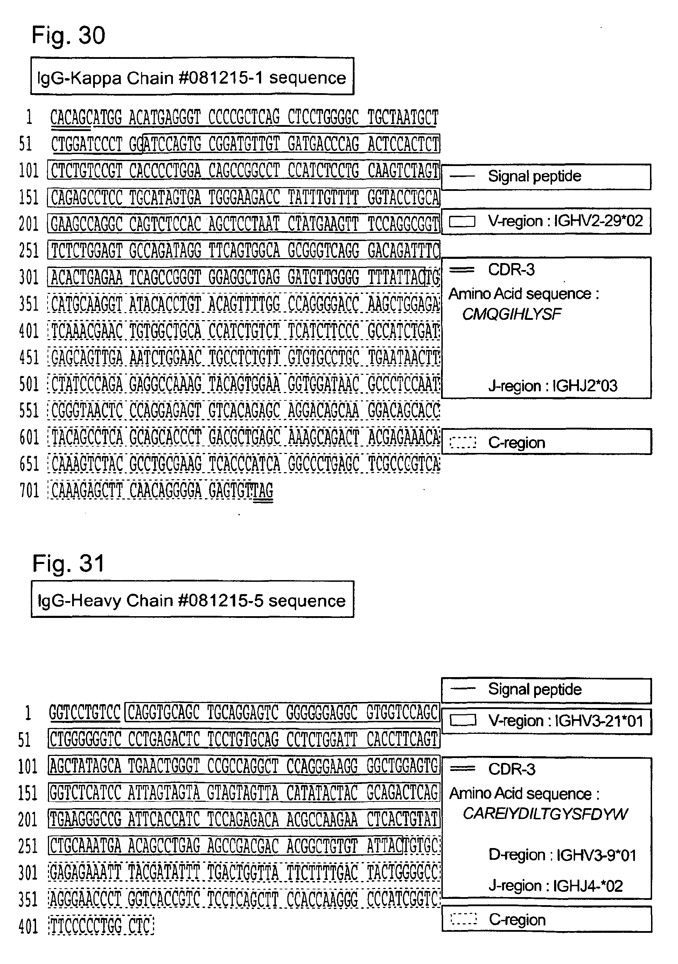

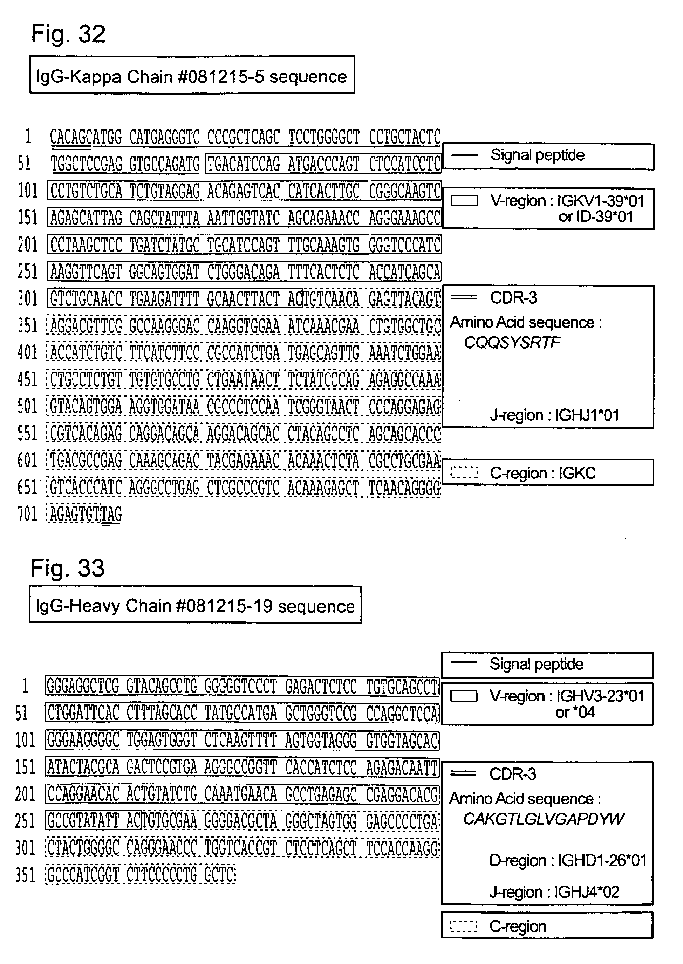

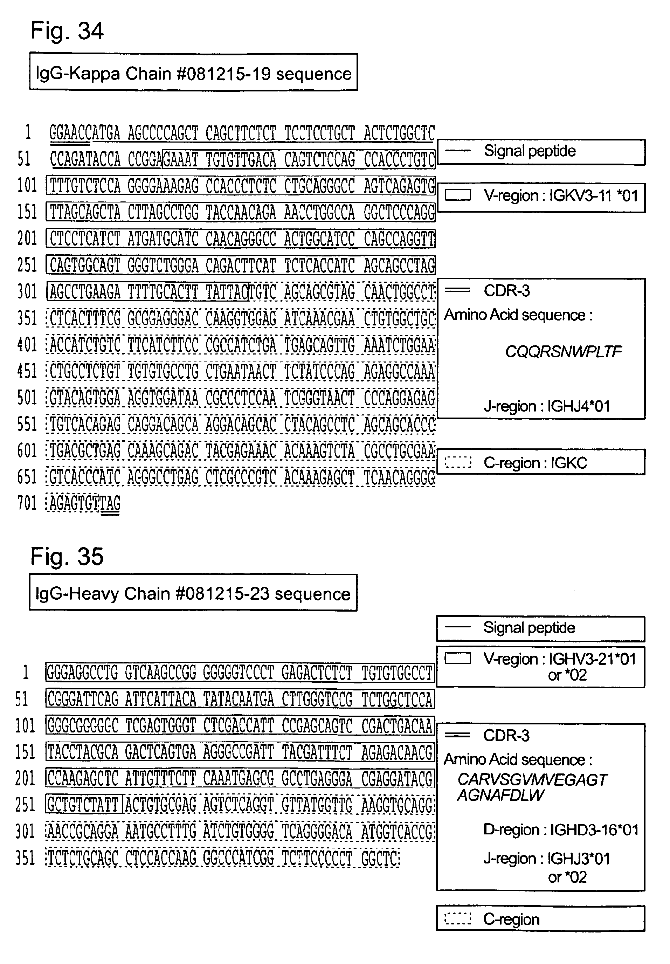

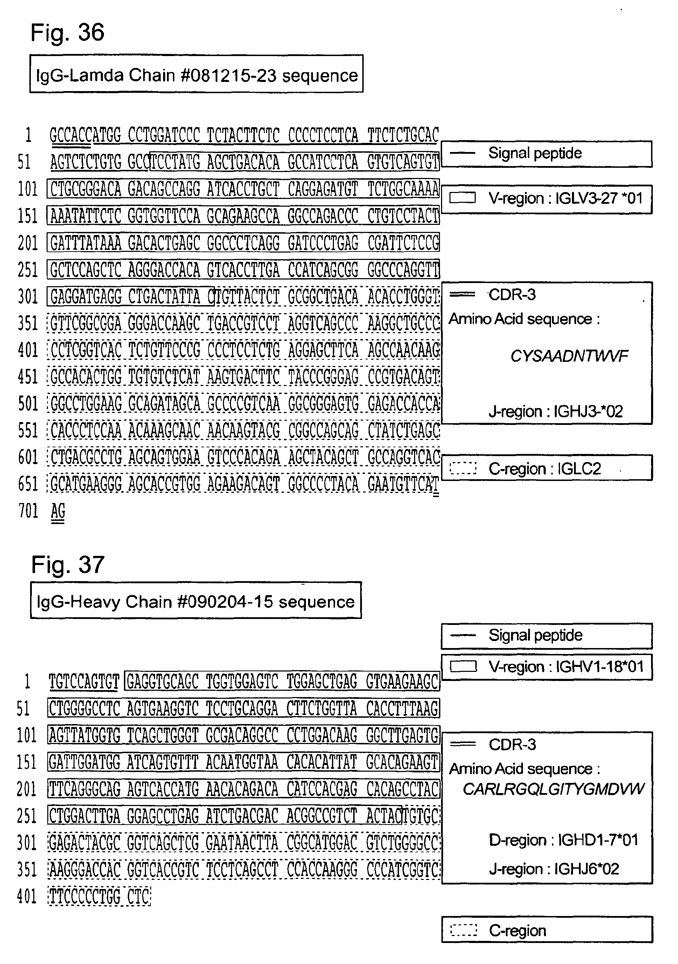

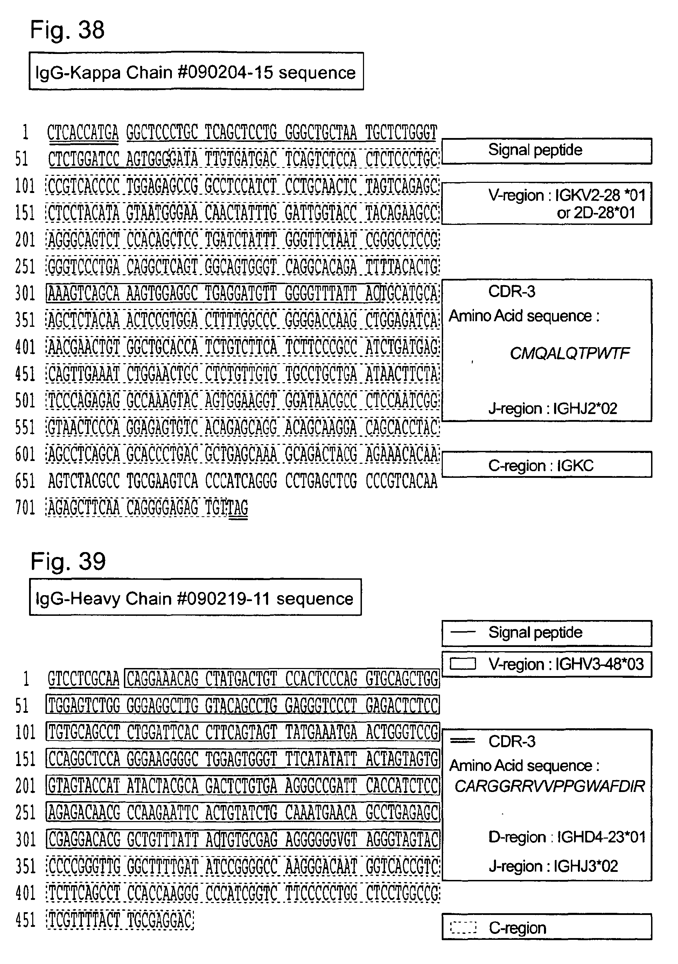

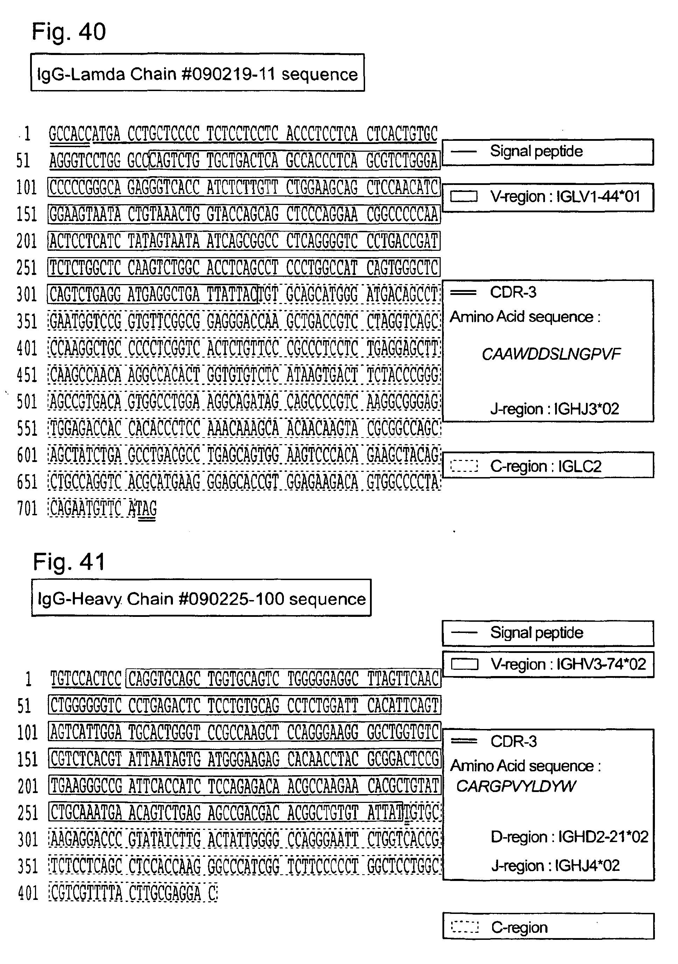

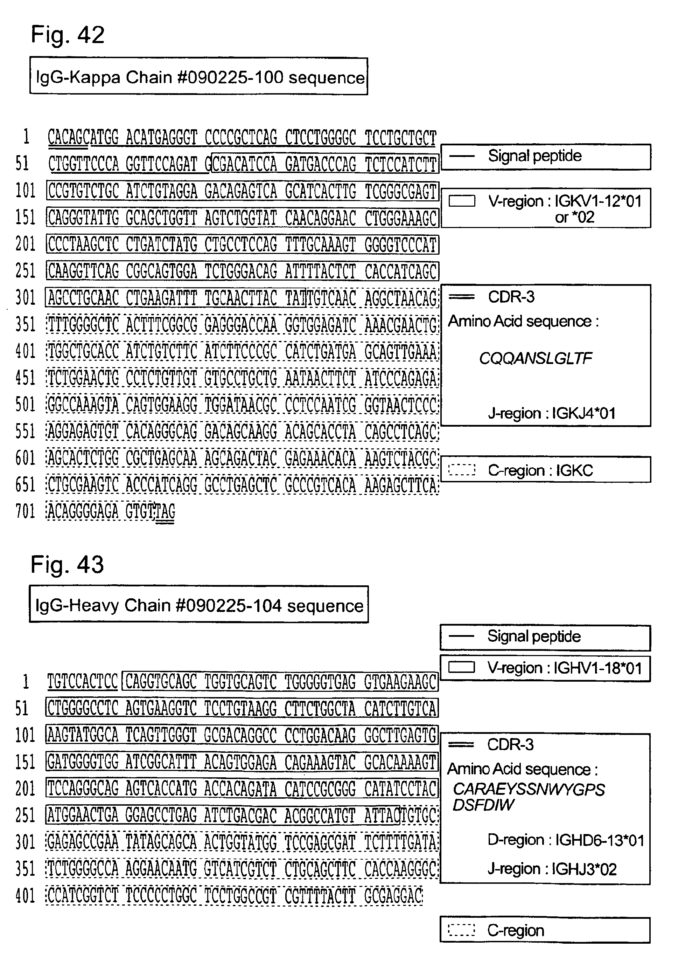

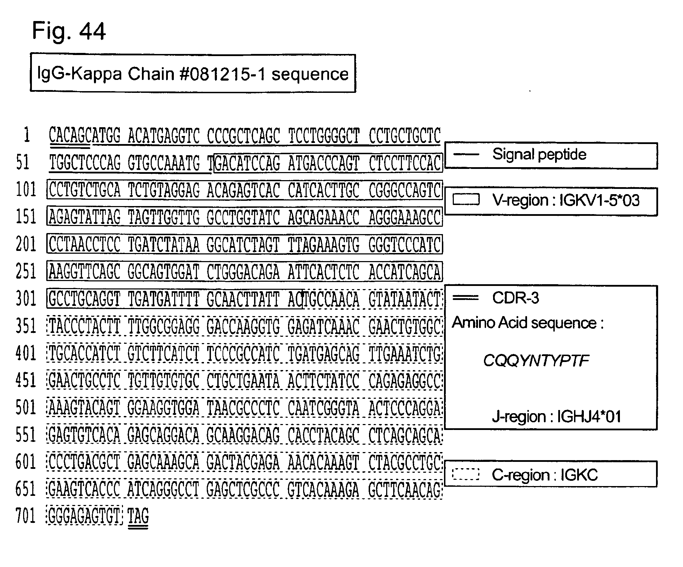

The results of sequence analysis of the repertoire, CDR-3 and full length of CMVpp65 antigen-specific IgG antibody genes are shown in Figures 27 and 30 to 44. Functional sequences were identified in 8 of the total 10 clones in which both antibody genes of IGH/L were successfully cloned. Six were derived by the single cell sorting method, and two were B cells derived by cell microarray. In the analysis of the repertoire used, the IGH and IGL used were all those from different types of families (Figure 27). The full-length sequences of IGH and IGL in each cloned cDNA are shown in Figures 29 to 44 and SEQ ID NOS: 71 to 86.

## Claims

1. A method for analyzing/identifying an antibody gene in one B cell derived from a human, successively comprising the steps of (A), (B), (C), (D), (E), (F) and (G), wherein the human is a cancer-bearing patient in whom a vaccine therapy has not been established, wherein the vaccine therapy increases B cells producing an antibody to a cancer-specific peptide to a certain percentage or more:
(A) harvesting peripheral blood mononuclear cells from peripheral blood obtained from the human;
(B) producing an immortalized B cell (EBV-B cell) line from the obtained peripheral blood mononuclear cells using Epstein-Barr virus (EBV);
(C) labeling the EBV-B cells with a marker-labeled antigen and with an antibody which is capable of recognizing a human antibody and is labeled with a marker different from the marker, wherein the antigen is a cancer-specific antigen peptide or cancer-specific antigen protein for a cancer of the cancer-bearing patient;
(D) separating EBV-B cells, that express an antibody recognizing the antigen on the cell membrane, on one cell by one cell basis;
(E) extracting total RNA from the one cell and synthesizing cDNA by reverse transcription reaction;
(F) using the synthesized cDNA as a template to perform a PCR reaction using a pair of primers specific for a human antibody heavy chain region gene, a PCR reaction using a pair of primers specific for a human antibody light chain κ region gene, or a PCR reaction using a pair of primers specific for a human antibody light chain λ region gene to amplify each of the region gene fragments; and
(G) analyzing/determining the base sequence of the amplified gene fragment.

2. The analyzing/identifying method according to claim 1, wherein the cancer-specific antigen peptide or cancer-specific antigen protein is MAGE1, MAGE2, MAGE3, MART1, tyrosinase, or gp100.

3. A method for preparing an antibody gene of one B cell derived from a human, successively comprising the steps of (A), (B), (C), (D), (E) and (F), wherein the human is a cancer-bearing patient in whom a vaccine therapy has not been established, wherein the vaccine therapy increases B cells producing an antibody to a cancer-specific peptide to a certain percentage or more:
(A) harvesting peripheral blood mononuclear cells from peripheral blood obtained from the human;
(B) producing an immortalized B cell (EBV-B cell) line from the obtained peripheral blood mononuclear cells using Epstein-Barr virus (EBV);
(C) labeling the EBV-B cells with a marker-labeled antigen and with an antibody which is capable of recognizing a human antibody and is labeled with a marker different from the marker, wherein the antigen is a cancer-specific antigen peptide or cancer-specific antigen protein for a cancer of the cancer-bearing patient;
(D) separating EBV-B cells, that express an antibody recognizing the antigen on the cell membrane, on one cell by one cell basis;
(E) extracting total RNA from the one cell and synthesizing cDNA by reverse transcription reaction; and
(F) using the synthesized cDNA as a template to perform a PCR reaction using a pair of primers specific for a human antibody heavy chain region gene, a PCR reaction using a pair of primers specific for a human antibody light chain κ region gene, or a PCR reaction using a pair of primers specific for a human antibody light chain λ region gene to amplify each of the region gene fragments.

4. The method for preparing an antibody gene according to claim 3, wherein the cancer-specific antigen peptide or cancer-specific antigen protein is MAGE1, MAGE2, MAGE3, MART1, tyrosinase, or gp100.

5. A method for producing an antibody of one B cell derived from a human, comprising preparing an antibody gene according to the method of claim 3 or 4 and expressing the antibody gene using an expression vector.

## Patentansprüche

1. Verfahren zur Analyse/Identifizierung eines Antikörpergens in einer menschlichen B-Zelle, das nacheinander die Schritte (A), (B), (C), (D), (E), (F) und (G) aufweist, wobei es sich bei dem Menschen um einen Krebspatienten handelt, bei dem keine Impftherapie durchgeführt wurde, wobei die Impftherapie B-Zellen erhöht, die bis zu einem bestimmten Prozentsatz oder mehr einen Antikörper gegen ein krebsspezifisches Peptid produzieren:
(A) Gewinnen von peripheren, mononukleären Blutzellen aus dem peripheren Blut eines Menschen;
(B) Produzieren einer immortalisierten B-Zelllinie (EBV-B Zelle) aus den gewonnenen peripheren, mononukleären Blutzellen unter Verwendung des Epstein-Barr Virus (EBV);
(C) Markieren der EBV-B Zellen mit einem mit einem Marker markierten Antigen und mit einem Antikörper, der einen menschlichen Antikörper erkennen kann und mit einem Marker markiert ist, der sich von dem Marker unterscheidet, wobei das Antigen ein krebsspezifisches Antigenpeptid oder ein krebsspezifisches Antigenprotein für einen Krebs des Krebspatienten ist;
(D) Trennen von EBV-B Zellen, die einen Antikörper exprimieren, der das Antigen an der Zellmembran erkennt, und zwar auf einer Basis Zelle für Zelle;
(E) Extrahieren der Gesamt-RNA aus der einen Zelle und Synthetisieren der cDNA durch reverse Transkriptionsreaktion;
(F) Verwenden der synthetisierten cDNA als Matrize zur Durchführung einer PCR Reaktion unter Verwendung eine Paares von Primern, die für eine Genregion von schweren Ketten eines menschlichen Antikörpers spezifisch sind, wobei eine PCR Reaktion ein Paar Primer verwendet, die für eine κ Genregion von leichten Ketten eines menschlichen Antikörpers spezifisch sind, oder eine PCR Reaktion ein Paar von Primem verwendet, die für eine λ Genregion von leichten Ketten eines menschlichen Antikörpers spezifisch sind, um jedes der Genregionfragmente zu amplifizieren; und
(G) Analysieren/Bestimmen der Basissequenz des amplifizierten Genfragmentes.

2. Analyse-/Identifizierungsverfahren nach Anspruch 1, wobei das krebsspezifische Antigenpeptid oder das krebsspezifische Antigenprotein MAGE1, MAGE2, MAGE 3, MART1, Tyrosinase oder gp100 ist.

3. Verfahren zur Herstellung eines Antikörpergens einer menschlichen B-Zelle, das nacheinander die Schritte (A), (B), (C), (D), (E) und (F) aufweist, wobei es sich bei dem Menschen um einen Krebspatienten handelt, bei dem keine Impftherapie durchgeführt wurde, wobei die Impftherapie B-Zellen erhöht, die bis zu einem bestimmten Prozentsatz oder mehr einen Antikörper gegen ein krebsspezifisches Peptid produzieren:
(A) Gewinnen von peripheren, mononukleären Blutzellen aus dem peripheren Blut eines Menschen;
(B) Produzieren einer immortalisierten B-Zelllinie (EBV-B Zelle) aus den gewonnenen peripheren, mononukleären Blutzellen unter Verwendung des Epstein-Barr Virus (EBV);
(C) Markieren der EBV-B Zellen mit einem mit einem Marker markierten Antigen und mit einem Antikörper, der einen menschlichen Antikörper erkennen kann und mit einem Marker markiert ist, der sich von dem Marker unterscheidet, wobei das Antigen ein krebsspezifisches Antigenpeptid oder ein krebsspezifisches Antigenprotein für einen Krebs des Krebspatienten ist;
(D) Trennen von EBV-B Zellen, die einen Antikörper exprimieren, der das Antigen an der Zellmembran erkennt, und zwar auf einer Basis Zelle für Zelle;
(E) Extrahieren der Gesamt-RNA aus der einen Zelle und Synthetisieren der cDNA durch reverse Transkriptionsreaktion; und
(F) Verwenden der synthetisierten cDNA als Matrize zur Durchführung einer PCR Reaktion unter Verwendung eine Paares von Primern, die für eine Genregion von schweren Ketten eines menschlichen Antikörpers spezifisch sind, wobei eine PCR Reaktion ein Paar Primer verwendet, die für eine κ Genregion von leichten Ketten eines menschlichen Antikörpers spezifisch sind, oder eine PCR Reaktion ein Paar von Primem verwendet, die für eine λ Genregion von leichten Ketten eines menschlichen Antikörpers spezifisch sind, um jedes der Genregionfragmente zu amplifizieren.

4. Verfahren zur Herstellung eines Antikörpergens nach Anspruch 3, wobei das krebsspezifische Antigenpeptid oder das krebsspezifische Antigenprotein MAGE1, MAGE2, MAGE3, MART1, Tyrosinase oder gp100 ist.

5. Verfahren zur Herstellung eines Antikörpers einer menschlichen B-Zelle, das die Herstellung eines Antikörpergens nach dem Verfahren der Ansprüche 3 oder 4 aufweist, und das Antikörpergen unter Verwendung eines Expressionsvektors exprimiert.

## Revendications

1. Procédé d'analyse/d'identification d'un gène d'anticorps dans une cellule B dérivée d'un humain, comprenant successivement les étapes (A), (B), (C), (D), (E), (F) et (G), dans lequel l'humain est un patient porteur d'un cancer dans lequel aucune thérapie vaccinale n'a été établie, dans lequel la thérapie vaccinale augmente la production par les cellules B d'un anticorps dirigé contre un peptide spécifique du cancer selon un certain pourcentage ou plus :
(A) récolter des cellules mononucléaires du sang périphérique à partir de sang périphérique obtenu de l'humain ;
(B) produire une lignée de cellules B immortalisées (cellules EBV-B) à partir de cellules mononucléaires de sang périphérique obtenues en utilisant le virus d'Epstein-Barr (EBV) ;
(C) marquer les cellules EBV-B avec un antigène marqué par un marqueur et avec un anticorps qui est capable de reconnaître un anticorps humain et marqué avec un marqueur différent du marqueur, l'antigène étant un peptide d'antigène spécifique du cancer ou une protéine d'antigène spécifique du cancer pour un cancer du patient porteur du cancer ;
(D) séparer les cellules EBV-B qui expriment un anticorps reconnaissant l'antigène sur la membrane cellulaire, en procédant cellule par cellule ;
(E) extraire l'ARN total d'une cellule et synthétiser l'ADNc par réaction de transcriptase inverse ;
(F) utiliser l'ADNc synthétisé comme matrice pour réaliser une réaction de PCR en utilisant une paire d'amorces spécifiques d'un gène de région de chaîne lourde d'un anticorps humain, une réaction de PCR en utilisant une paire d'amorces spécifiques d'un gène de région κ de chaîne légère d'un anticorps humain, ou une réaction de PCR en utilisant une paire d'amorces spécifique d'un gène de région λ de chaîne légère d'un anticorps humain pour amplifier chacun des fragments de gène de la région ; et
(G) analyser/déterminer la séquence de base du fragment de gène amplifié.

2. Procédé d'analyse/d'identification selon la revendication 1, dans lequel le peptide d'antigène spécifique du cancer ou la protéine d'antigène spécifique du cancer est MAGE1, MAGE2, MAGE3, MART1, la tyrosinase ou gp100.

3. Procédé de préparation d'un gène d'anticorps d'une cellule B dérivée d'un humain, comprenant successivement les étapes (A), (B), (C), (D), (E) et (F), dans lequel l'humain est un patient porteur d'un cancer dans lequel aucune thérapie vaccinale n'a été établie, dans lequel la thérapie vaccinale augmente la production par les cellules B d'un anticorps dirigé contre un peptide spécifique du cancer selon un certain pourcentage ou plus :
(A) récolter des cellules mononucléaires du sang périphérique à partir de sang périphérique obtenu de l'humain ;
(B) produire une lignée de cellules B immortalisées (cellules EBV-B) à partir de cellules mononucléaires de sang périphérique obtenues en utilisant le virus d'Epstein-Barr (EBV) ;
(C) marquer les cellules EBV-B avec un antigène marqué par un marqueur et avec un anticorps qui est capable de reconnaître un anticorps humain et est marqué avec un marqueur différent du marqueur, l'antigène étant un peptide d'antigène spécifique du cancer ou une protéine d'antigène spécifique du cancer pour un cancer du patient porteur du cancer ;
(D) séparer les cellules EBV-B qui expriment un anticorps reconnaissant l'antigène sur la membrane cellulaire, en procédant cellule par cellule ;
(E) extraire l'ARN total d'une cellule et synthétiser l'ADNc par réaction de transcriptase inverse ; et
(F) utiliser l'ADNc synthétisé comme matrice pour réaliser une réaction de PCR en utilisant une paire d'amorces spécifiques d'un gène de région de chaîne lourde d'un anticorps humain, une réaction de PCR en utilisant une paire d'amorces spécifiques d'un gène de région κ de chaîne légère d'un anticorps humain, ou une réaction de PCR en utilisant une paire d'amorces spécifique d'un gène de région λ de chaîne légère d'un anticorps humain pour amplifier chacun des fragments de gène de la région.

4. Procédé de préparation d'un gène d'anticorps selon la revendication 3, dans lequel le peptide d'antigène spécifique du cancer ou la protéine d'antigène spécifique du cancer est MAGE1, MAGE2, MAGE3, MART1, la tyrosinase ou gp100.

5. Procédé de production d'un anticorps d'une cellule B dérivée d'un humain, comprenant la préparation d'un gène d'anticorps selon le procédé de la revendication 3 ou 4 et l'expression du gène d'anticorps en utilisant un vecteur d'expression.
